(19) Europäisches Patentamt
European Patent Office
Office européen des brevets

(11) Numéro de publication: **0 117 961 B1**

(12) # FASCICULE DE BREVET EUROPEEN

(45) Date de publication de fascicule du brevet: **03.04.91**

(21) Numéro de dépôt: **83402549.6**

(22) Date de dépôt: **28.12.83**

(51) Int. Cl.5: **C07D 233/46**, C07D 233/48, C07D 233/50, C07D 233/52, C07D 405/04, C07D 405/12, C07D 233/22, C07D 239/06, C07D 277/40, A61K 31/33

(54) **Nouveaux dérivés hétérocycliques substitués par un radical aminé, leurs procédé d'obtention et les compositions pharmaceutiques en renfermant.**

(30) Priorité: **28.12.82 CH 7591/82**

(43) Date de publication de la demande: **12.09.84 Bulletin 84/37**

(45) Mention de la délivrance du brevet: **03.04.91 Bulletin 91/14**

(84) Etats contractants désignés: **AT BE CH DE FR GB IT LI LU NL SE**

(56) Documents cités: **FR-A- 2 448 534** **US-A- 3 147 270**

(73) Titulaire: **Albert ROLLAND S.A. Société dite**
**49, Rue St-André-des-Arts**
**F-75006 Paris(FR)**

(72) Inventeur: **Moinet, Gerard**
**15 Rue Lamartine**
**F-91400 Orsay(FR)**
Inventeur: **Schaefer, Michel**
**4 Rue François Girardon**
**F-91380 Chilly-Mazarin(FR)**
Inventeur: **Bessin, Pierre**
**1 Allée Bossuet**
**F-91380 Chilly-Mazarin(FR)**
Inventeur: **Bonnet, Jacqueline**
**19 Rue Charcot**
**F-75013 Paris(FR)**

(74) Mandataire: **Burtin, Jean-François**
**Cabinet GEFIB 55 Rue Boissonade**
**F-75014 Paris(FR)**

EP 0 117 961 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention a pour objet de nouveaux dérivés hétérocycliques porteurs d'un groupe aminé ainsi que des procédés pour leur obtention. L'invention a plus particulièrement pour objet de nouveaux composés hétérocycliques dont la structure cyclique comporte deux hétéroatomes, identiques ou différents, substitués par un groupe amino libre ou substitué.

L'invention a spécifiquement pour objet les composés répondant à la formule générale I

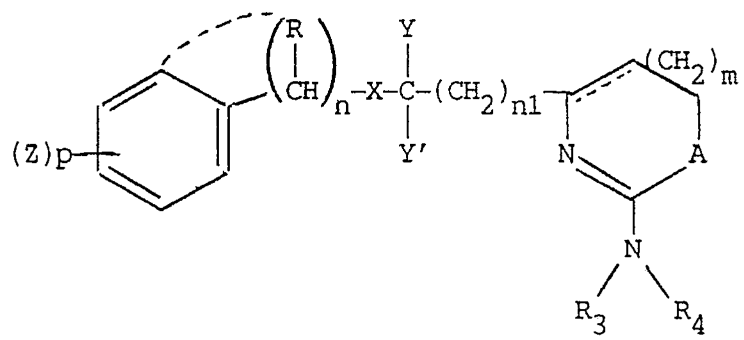

(I)

dans laquelle Z est de l'hydrogène, un atome d'halogène, un radical alcoyle inférieur, un radical alcoxy inférieur, un radical trifluorométhyle, un radical trifluoro méthoxy, cyano, nitro, carboxamido, un radical alcényle inférieur, un radical (alcoyle inférieur) thio, un groupe alcoylène dioxy, un radical cycloalcényle ou cycloalcoyle inférieurs

X représente de l'oxygène, du soufre, un radical imino de formule > NH un radical méthylène ou une liaison directe

Y représente de l'hydrogène, un radical alcoyle inférieur, un radical phényle, un hydroxyle ou un radical phénoxy

Y' représente de l'hydrogène

ou bien Y et Y' forment ensemble l'oxygène d'un groupe carbonyle

ou bien Y forme avec le radical phényle adjacent lorsque n est égal à zéro, une structure bicyclique hétérocyclique choisie dans le groupe constitué par le tétrahydroindole, le dihydrobenzothiophène et le thiochromane

A représente un groupe NH ou du soufre

$R_3$ et $R_4$, semblables ou différents, représentent de l'hydrogène, un radical alcoyle inférieur, alcényle inférieur, aralcoyle inférieur dont la chaine hydrocarbonée a de 1 à 6 atomes de carbone ou (hétéroaryle) alcoyle, ayant de 1 à 6 atomes de carbone dans la chaine hydrocarbonée, alcoyloxy carbonyle, acyle dérivé d'un acide organique carboxylique ayant de 1 à 10 atomes de carbone ou un radical amino

ou bien $R_3$ et $R_4$ forment avec l'atome d'azote auxquels ils sont liés, la chaine alcoylène d'une structure hétérocyclique azotée, éventuellement interrompue par un ou deux autres hétéroatomes

R représente un hydrogène, un radical alcoyle inférieur ayant de 1 à 6 atomes de carbone, un radical phénylène relié au cycle benzénique par une chaine alcoylène ayant 1 ou 2 atomes de carbone

n est étal à zéro ou 1

$n_1$ est égal à 0, 1 ou 2

m est égal à 0 ou 1

et p est égal à 1, 2 ou 3 le trait pointillé symbolise une double liaison carbone-carbone éventuelle.

L'invention se rapporte aussi aux sels des composés de formule générale I avec un acide minéral ou organique, de préférence thérapeutiquement compatible.

L'invention comprend également les formes tautomères imino des composés de formule géérale I. En effet, par suite de la délocalisation de la double liaison intracyclique du cycle azoté, les composés de formule générale I dans laquelle au moins un des substituants $R_3$ et $R_4$ représente de l'hydrogène peuvent exister sous forme de dérivé imino répondant à la formule générale I'

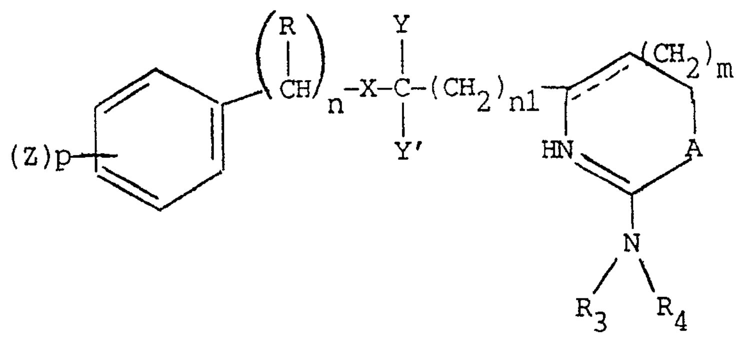

(I')

dans laquelle la définition des substituants Z, p, R, X, Y, Y', $R_4$, n, $n_1$ et m demeure celle fournie précédemment.

La forme amino I et la forme imino I' peuvent exister simultanément ou bien les deux formes peuvent être obtenues distinctement soit sous forme libre, soit sous forme salifiée.

L'invention s'étend encore aux formes optiquement actives des composés de formule générale I, qui comportent au moins un atome chiral, notamment lorsque les susbtituants Y et Y' sont différents. Parmi les composés de formule générale I, on distinguera plus particulièrement les groupes suivants :

1) les dérivés de formule générale I"

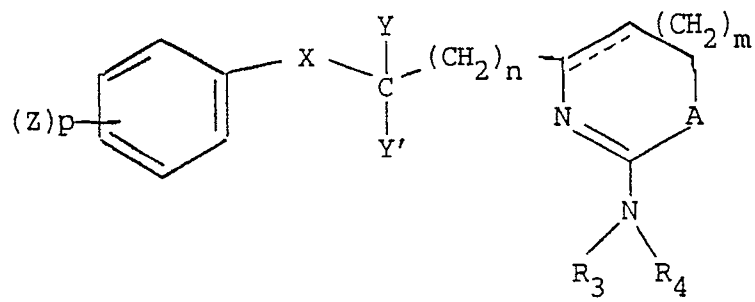

(I")

qui correspondent au cas où n est égal à zéro et dans laquelle X représente de l'oxygène, du soufre, un radical imino de formule $NR_1$ dans laquelle $R_1$ est de l'hydrogène, un radical méthylène ou une liaison directe.

2) Les composés de formule générale I'''

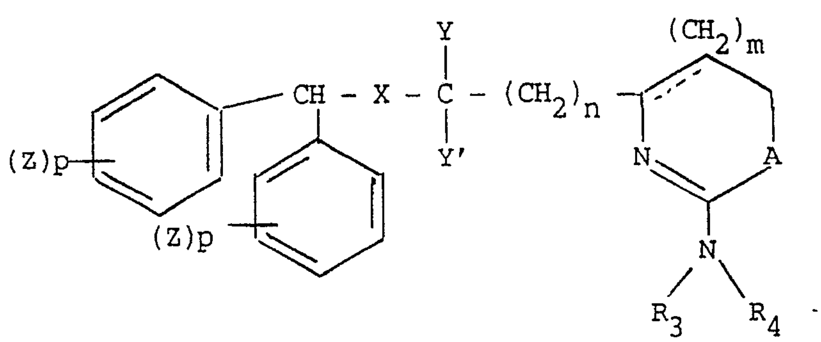

(I''')

dans laquelle la définition des substituants demeure celle fournie pour les composés de formule générale I

3) Les composés de formule générale I""

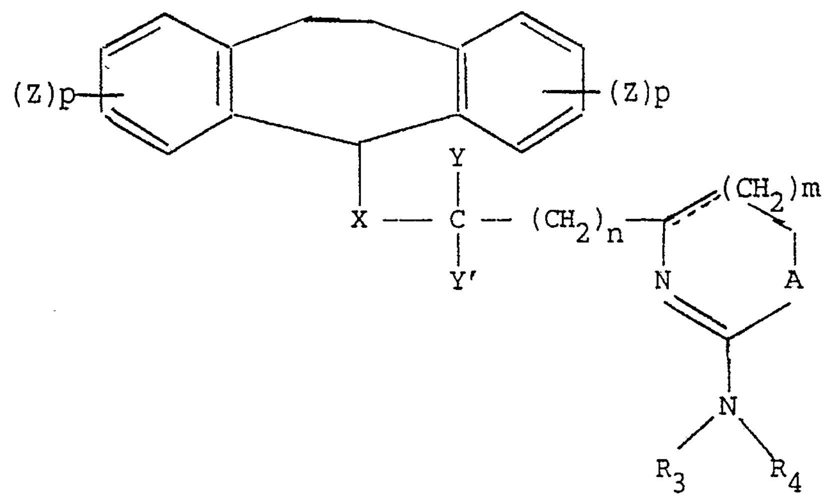

(I"")

dans laquelle la définition des substituants Z, X, Y, Y', $R_3$, $R_4$, A, m, n et p demeure celle fournie pour les composés de formule générale I et plus particulièrement les sous-groupes de formule générale

1. Les imidazolines de formule générale $I_A$

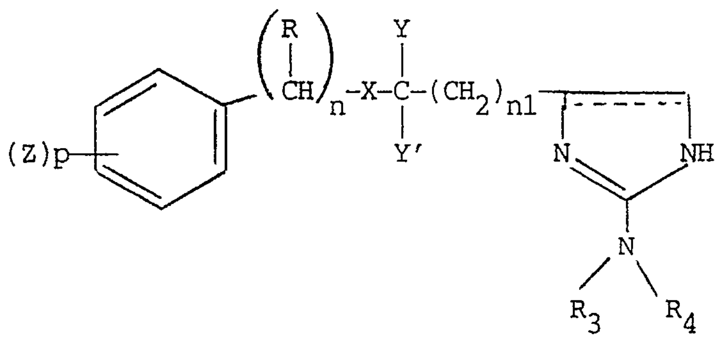

($I_A$)

dans laquelle les substituants X, Y, Y', Z, R, $R_3$, $R_4$, n, $n_1$ et p ont les significations fournies précédemment et m est égal à 0

2. Les tétrahydropyrimidines de formule générale $I_B$

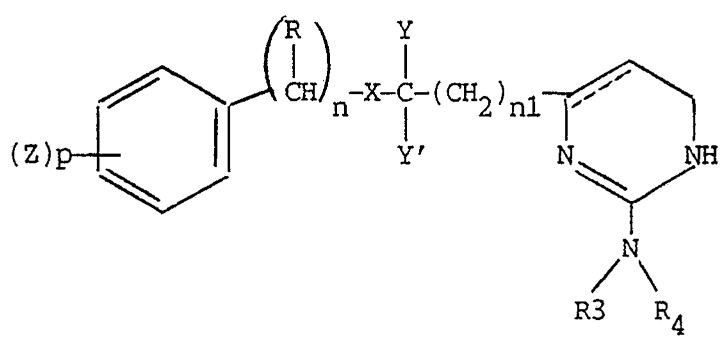

($I_B$)

dans laquelle les substituants X, Y, Y', Z, $R_3$, $R_4$, n et p ont les définitions fournies antérieurement et m est égal à 1.

3. Les 2-aminothiazoles de formule générale $I_C$

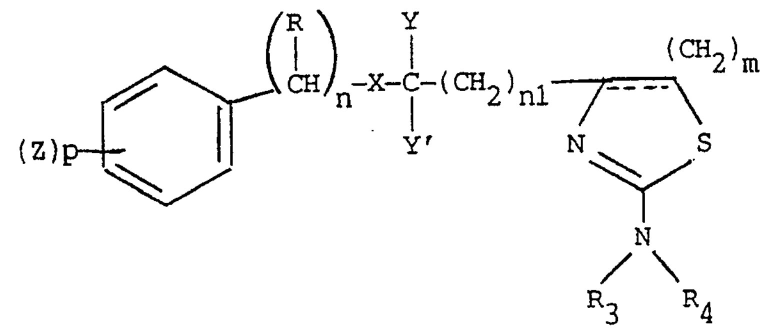

($I_C$)

dans laquelle les substituants X, Y, Y', Z, R, $R_3$, $R_4$, n, $n_1$ et p ont les définitions fournies antérieurement ainsi que les sels de ces composés avec un acide minéral ou organique et en particulier, les composés de formule générale $I_D$

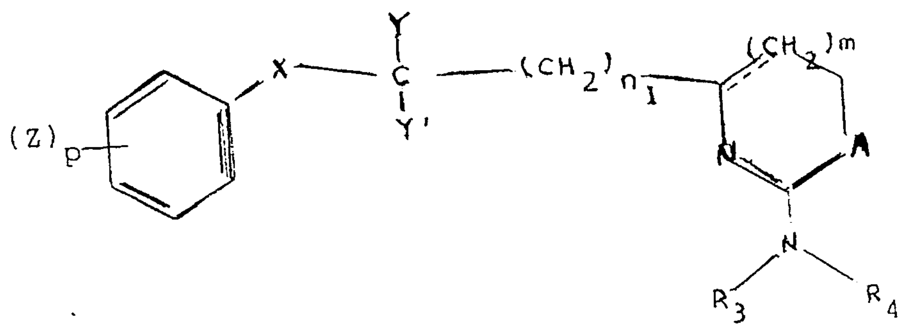

($I_D$)

dans laquelle Z, X, Y, Y', $R_3$, $R_4$, A, n, m et p ont les définitions antérieures.

Parmi les acides physiologiquement compatibles, on pourra citer les chlorhydrates, bromhydrates, les sulfates, les nitrates, les phosphates, les sulfites, les acétates, les butyrates, les caproates, les subérates, les succinates, les tartrates, les citrates, les itaconates, les glutamates, les aspartates, les benzoates, les triméthoxybenzoates, les salicylates, les niflumates, les flufénamates, les méfénamates, les nicotinates, les isonicotinates, les benzène-sulfonates, les méthanesulfonates, les éthanesulfonates, les iséthionates, les paratoluènesulfonates, les naphtalènesulfonates, les glucose 1-phosphates ou les glucoses 1,6-diphosphates.

Les acides qui ne sot pas utilisables en thérapeutique peuvent servir comme moyen d'isolement, de purification ou de dédoublement.

On pourra citer, à cet égard, les perchlorates, les iodates, les bromates, les vanadates ou les chromates ; les sels avec l'acide strychnique, l'acide d-chrysanthémique, l'acide indolyl-3 acétique, l'acide dichlorophénoxy-isobutyrique ou l'acide citraconique.

Parmi les acides physiologiquement compatibles, on pourra citer les chlorhydrates, bromhydrates, les sulfates, les nitrates, les phosphates, les sulfites, les acétates, les butyrates, les caproates, les subérates, les succinates, les tartrates, les citrates, les itaconates, les glutamates, les aspartates, les benzoates, les triméthoxybenzoates, les salicylates, les niflumates, les flufénamates, les méfénamates, les nicotinates, les isonicotinates, les benzènesulfonates, les méthanesulfonates, les éthanesulfonates, les iséthionates, les paratoluè-nesulfonates, les naphtalènesulfonates, les glucose 1-phosphates ou les glucoses 1, 6-diphosphates.

Les acides qui ne sont pas utilisables en thérapeutique peuvent servir comme moyen d'isolement, de purification ou de dédoublement.

On pourra citer à cet égard les perchlorates, les iodates, les bromates, les vanadates ou les chromates ; les sels avec l'acide strychnique, l'acide d-chrysanthémique, l'acide indolyl -3 acétique, l'acide dichlorophénoxy-isobutyrique ou l'acide citraconique.

Pour autant que l'invention soit concernée, le terme alcoyle inférieur désigne un radical hydrocarboné ayant de 1 à 6 atomes de carbone en chaîne linéaire ou ramifiée. Des exemples de tels radicaux sont le radical méthyle, éthyle, isopropyle, sec-butyle, ter butyle, neopentyle ou n-hexyle.

Un radical alcoxy inférieur comporte un radical alcoyle défini comme précèdemment.

Parmi les halogènes on citera plus particulièrement le fluor ou le chlore. Néanmoins, les dérivés

bromés et iodés sont d'un intérêt équivalent.

Un radical alcenyle inférieur est un radical hydrocarboné portant une double liaison, comportant de 2 à 6 atomes de carbone. On pourra citer à titre d'exemple de radicaux alcenyle le radical allyle, le radical methallyle, le radical but 2-enyle, le radical isopropényle ou le radical methyl-3 butl-enyle.

Un radical alcoylène dioxy comporte de 1 à 4 atomes dans la chaîne alcoylène comme par exemple le radical methylènedioxy ou éthylènedioxy.

Lorsque Y représente un radical phényle substitué, les substituants peuvent être de un à trois atomes d'halogène, un radical trifluorométhyle ou trifluoromethoxy, un à trois radicaux alcoyle inférieur ou alcoxy inférieur.

Lorsque Y forme avec le radical phényle adjacent une structure bicyclique, les composés formés répondent à la formule générale $I_E$

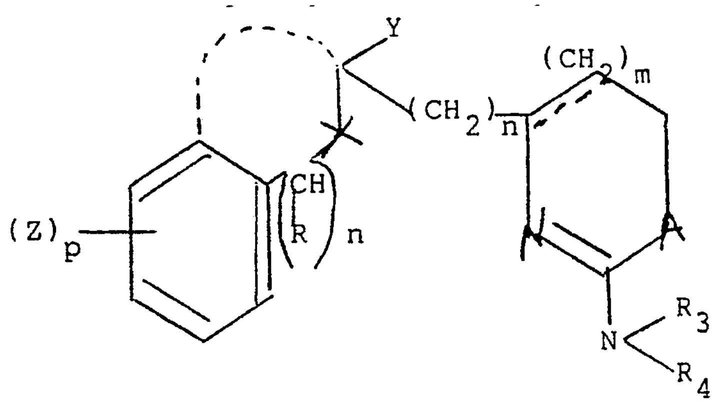

($I_E$)

dans laquelle X garde les significations fournies précédemment.
Y est de l'hydrogène ou un hydroxyle, les substituants Z, $R_3$ $R_4$, A, m, n et p sont définis comme ci-dessus.

On citera à titre d'exemple de telles structures bicycliques, les composés de formule générale

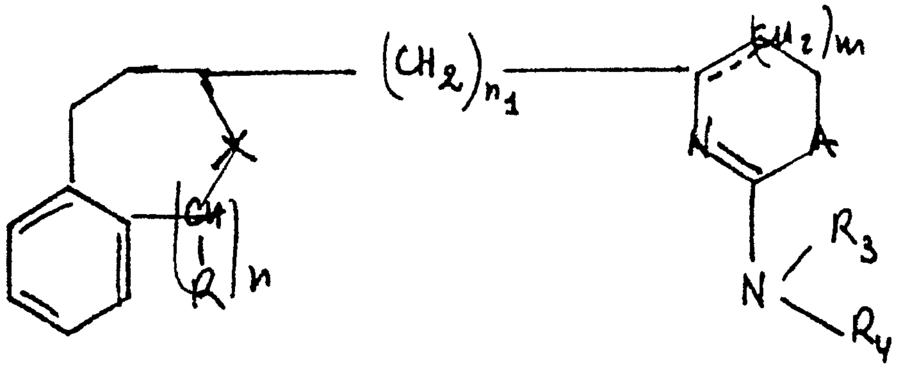

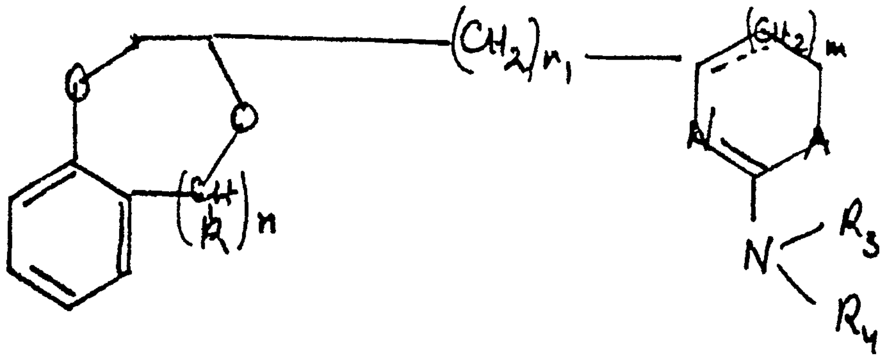

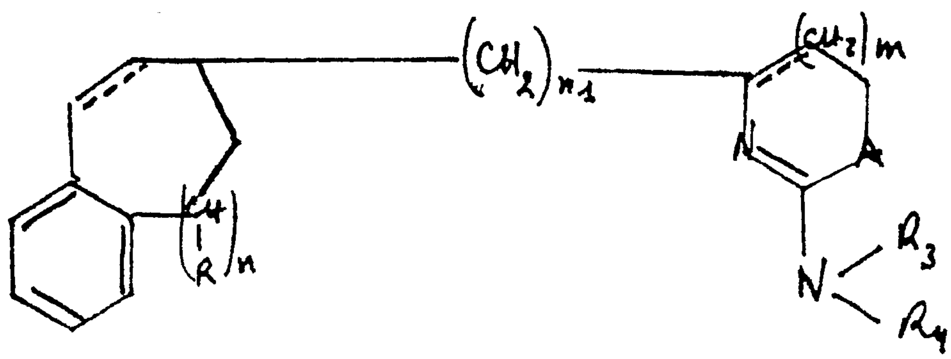

dans laquelle X représente 0, S ou N-$R_1$ et dans laquelle n est égal à 0 ou 1 - la structure bicyclique est un benzothiophène - un benzodioxan, une tétrahydroquinoléine, un tetrahydronaphtalène, un dihydronaphtalène ou un benzimidazole.

Ces cycles peuvent comporter des degrès d'insaturation supplémentaire, comme par exemple un naphtyle-1, ou naphtyle-2.

Un radical aralcoyle inférieur est un radical arylmonocyclique porteur d'une chaîne hydrocarboné ayant de 1 à 6 atomes de carbone en chaîne droite ou ramifiée. Des exemples de tels radicaux sont le radical benzyle, phenylethyle, $\alpha$ - methylpheny-lethyle, 2,6-dichlorobenzyle ou 2,3,5-trimethoxybenzyle. Un radical (hétéroaryle) alcoyle inférieur est un radical hétérocyclique aromatique porteur d'une chaîne hydrocarbonée ayant de 1 à 6 atomes de carbone. Des exemples de tel; radicaux sont par exemple le pyridyl-2 methyle ou le furyle ethyle, le pyranyl ethyle ou le thienyl -2methyle.

Lorsque $R_3$ et $R_4$ forment ensemble une chaîne alcoylène, la structure cyclique qui en résulte, comporte de 4 à 7 chaînons comme l'azétidine, la pyrrolidine, la pipéridine ou l'hexamethyléne imine. Lorsque cette chaîne est interrompue par un ou deux hétéro atomes, le cycle qui en résulte est par exemple une tétrahydropyrimidine, une tetrahydro oxazine, une morpholine une thiazine, une pyrazolidine ou une piperazine. Ces cycles peuvent porter des substituants comme par exemple des radicaux alcoyle inférieur, hydroxy alcoyle inférieur, pyridyle, phenyle non substitué ou substitué ou pyrimidyle.

Lorsque $R_3$ ou $R_4$ représentent un radical acyle, celui-ci peut dériver d'un acide aliphatique comme un radical acetyle, propionyle ou dipropylacetyl-ou d'un acide aromatique, comme un radical benzoyle, naphtoyl - 1, dichloro 2-6 benzoyle, trimethoxy 3, 4, 5 - benzoyle, veratroyle, syringoyle, O-carbethoxy syringoyle, nicotinoyle ou furoyle, ou d'un acide aryloxy alcanoïque comme l'acide phenoxy acétique, l'acide dichlorophenoxy acetique ou p-chlorophenoxy isobutyrique.

L'invention concerne aussi un procédé d'obtention des composés de formule générale I dans laquelle m est égal à 0 qui consiste en ce que l'on condense une epihalohydrine de formule générale II

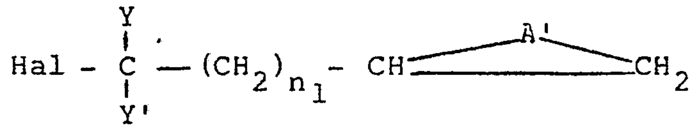

(II)

A' est de l'oxygène ou du soufre
dans laquelle Hal est du Chlore ou du Brome les substituants Y, Y' et $n_1$ ont les significations fournies antèrieurement avec un dérivé aromatique de formule générale III

(III)

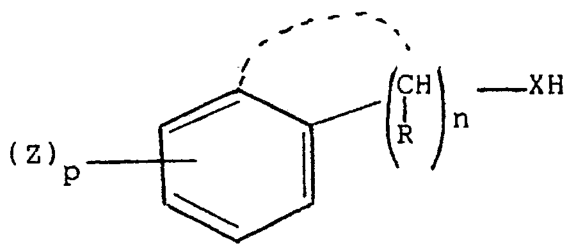

dans laquelle les substituants R, Z, n, p et X sont définis comme ci-dessus pour obtenir un dérivé aralcoylé de formule générale

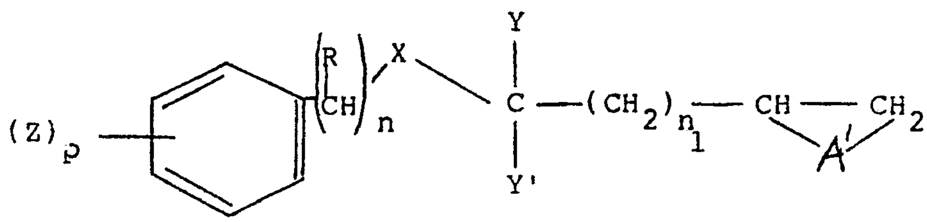

(IV)

IV dans laquelle la définition des substituants R, A', X, Y, Y', Z, n, $n_1$ et p demeure inchangée,
dont on ouvre le cycle oxirane par action d'un azidure de métal alcalin pour former un mono azide de formule générale V

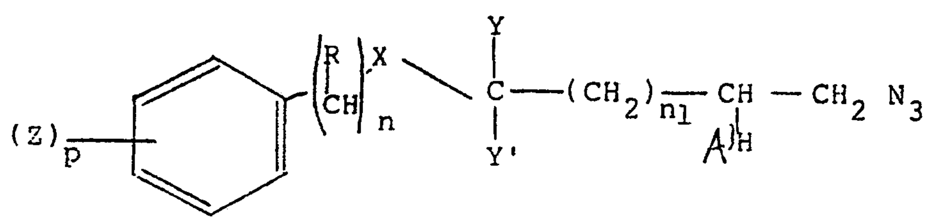

(V)

dans laquelle les substituants R, A', X, Y, Y' , Z, n, $n_1$ et p sont définis comme précédemment
soumet celui-ci à l'estérificationlorsque A' représente de l'oxygène à l'aide d'un dérivé fonctionnel d'acide sulfonique de formule générale VI

$$R_2\ SO_2\ R_5 \qquad (VI)$$

dans laquelle $R_2$ représente un radical alcoyle ou alcoyle substitué ou un radical aromatique mono- ou bicyclique et $R_5$ est un groupe alcoyle inférieur oxy ou un atome d'halogène en présence d'une base tertiaire, pour obtenir un azido ester de formule générale VII

(VII)

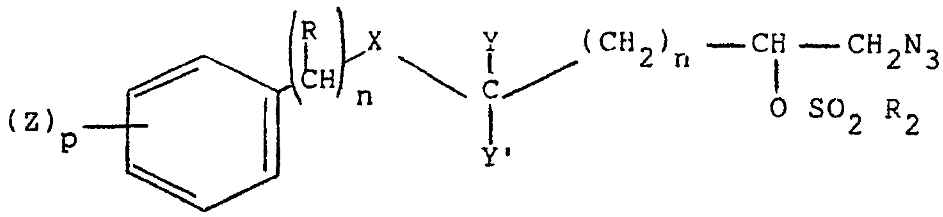

dans laquelle les substituants X, Y, Y', Z, n, p et $R_2$ sont définis comme précèdemment traite celui-ci par un azidure ou un thiocyanate de metal alcalin dans un solvant polaire pour former le dérivé azido de formule générale VIII

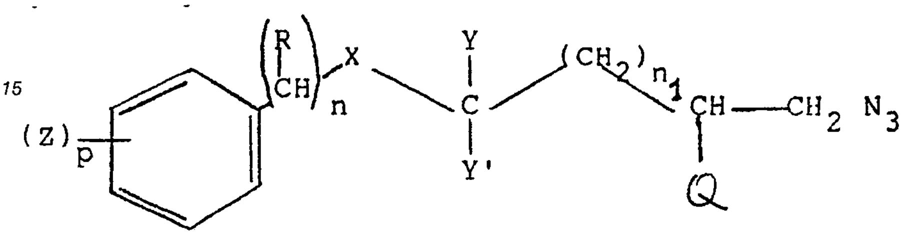

(VIII)

dans laquelle les définitions des substituants R X, Y, Y', Z, n $n_1$ et p demeurent inchangées et Q représente un radical SH ou azido que l'on réduit par hydrogénation en présence d'un catalyseur en aminoethane de formule générale IX

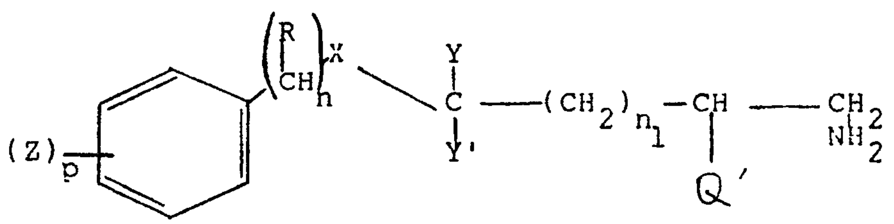

(IX)

dans laquelle les substituants R, X, Y, Y', Z, n, $n_1$ et p sont définis comme précèdemment et Q' est un radical SH ou amino, condense celui-ci avec un réactif de carboimination choisi dans le groupe constitué par les halogènures de Cyanogène et les halogènures de S-methyl Isothiouronium de formule générale

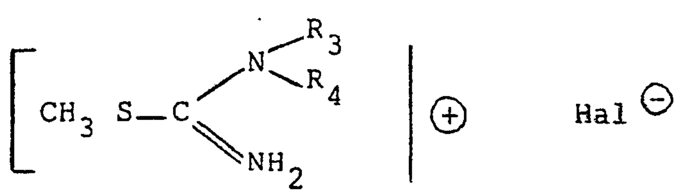

pour obtenir un composé de formule générale I dans laquelle R, X, Y, Y', Z, n, $n_1$ et p ont les significations fournies antérieurement
A représente du soufre ou un radical imino $R_3$ et $R_4$ représentent tous deux de l'hydrogène lorsque le réactif de carboimination est un halogènure de cyanogène ou $R_3$ et $R_4$ représentent un hydrogène et/ou un radical alcoyle inférieur, alcényle inférieur, aralcoyle inférieur, (hétéroaryl) alcoyle inférieur, aryle, alcoyloxy carbonyle ou acyle dérivé d'un acide organique carboxylique ayant de 1 à 10 atomes de carbone.
ou bien $R_3$ et $R_4$ forment avec l'atome d'azote une chaîne alcoylène, lorsque le réactif de carboimination est un halogènure de S-méthyl isothiouronium
que l'on peut, si désiré, salifier par addition d'un acide minéral ou organique ou dédoubler en ses isomères optiques par action d'un réactif chiral.

L'invention concerne également un procédé de préparation des composés de formule générale I, qui consiste à condenser un composé aromatique de formule générale V avec un halogènure d'allyle pour former un dérivé alcenylé de formule générale X

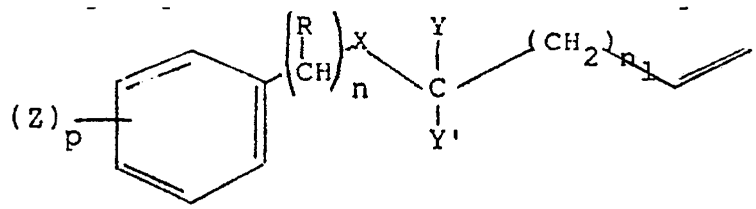

(X)

dans laquelle z, p, R, n, Y, Y' et $n_1$ sont définis comme précédemment
que l'on soumet à une bromation par action du brome pour former un dérivé dibromé de formule générale XI

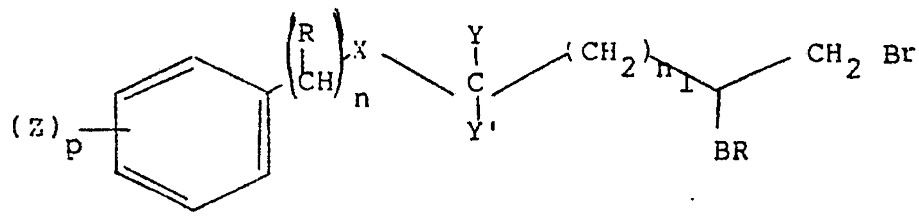

(XI)

puis fait réagir ce dernier avec un azidure de métal alcalin pour obtenir un diazide de formule générale VIII

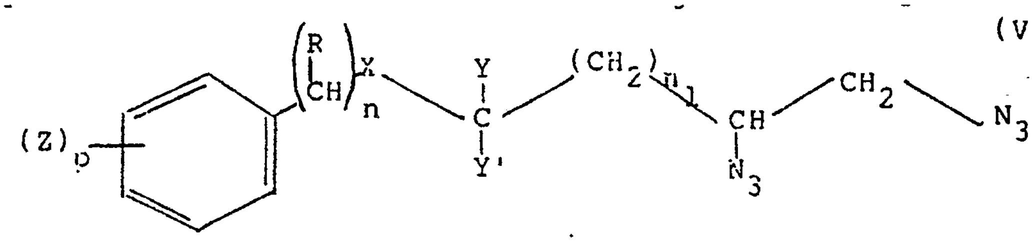

(VIII)

que l'on réduit en dérivé diaminé de formule générale IX par hydrogénation en présence d'un catalyseur qui fournit par cyclisation sous l'action d'un agent de carboimination comme un halogènure de cyanogène un dérivé imidazolique de formule générale $I_A$

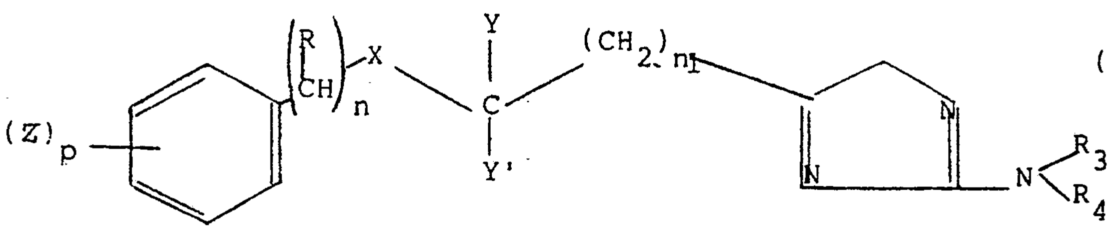

($I_A$)

dans laquelle la définition des substituants R, Z, X, Y, Y' , $R_3$, $R_4$, n, $n_1$ et p demeure inchangée

L'invention comprend encore un autre procédé de préparation des composés de formule générale I, qui consiste en ce que l'on condense une aryloxyalcoylaldehyde de formule générale XII

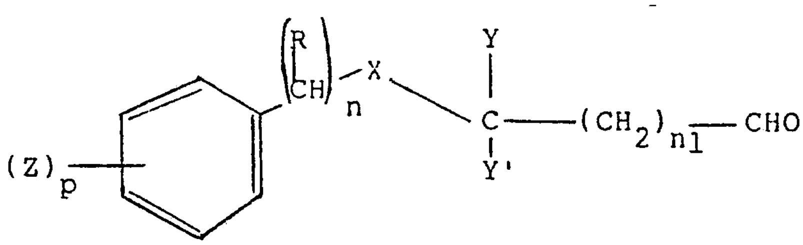

(XII)

dans laquelle la définition des substituants R X, Y, Y', Z, p et n demeure inchangée avec un cyanure de métal alcalin dans les conditions de la réaction de Strecker pour former une $\alpha$ -cyano amine de formule générale XIII

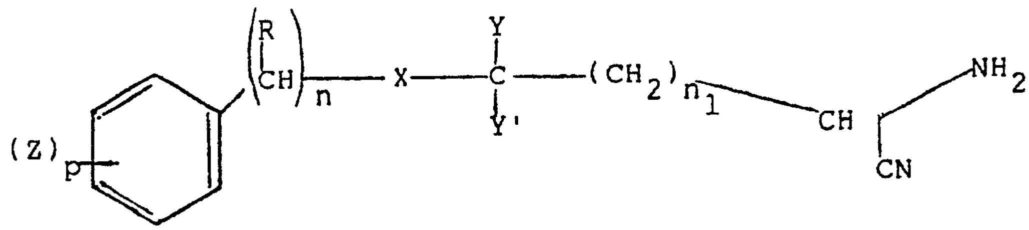

dans laquelle les substituants R, Z, Y, Y', n, $n_1$ et p sont définis comme précédemment qui est réduite par hydrogénation en présence d'un catalyseur en diaminoéthane substitué de formule générale IX

(IX)

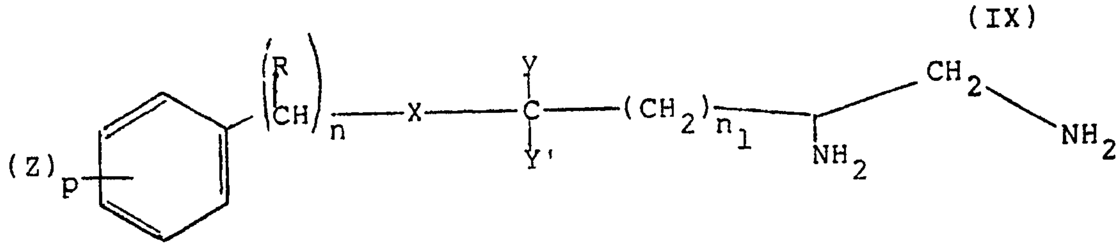

dans laquelle la définition des substituants R, X, Y,'Y', Z, n, $n_1$ et p demeure inchangée qui par condensation avec un réactif de carbo-imination fournit le dérivé cyclique de formule générale $I_A$.

Pour obtenir les composés de formule générale I dans laquelle $R_3$ et/ou $R_4$ sont un radical acyle, alcoyle ou alcenyle, ou aryle ou aralcoyle, on utilise un procédé qui consiste à condenser un diaminoethane de formule générale IX

(IX)

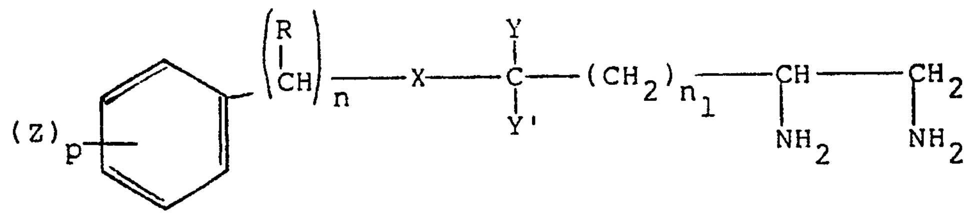

dans laquelle la définition des substituants Z, p, R, X, Y, Y', n et $n_1$ demeure celle fournie précédemment avec le sulfure de carbone, pour obtenir une imidazoline thione de formule générale XIV

(XIV)

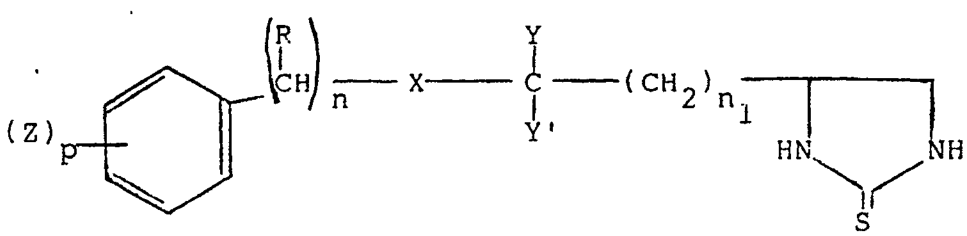

dans laquelle la définition des substituants Z, p, R, n, X, Y, Y' et $n_1$ demeure inchangée que l'on soumet à l'action d'un agent d'alcoylation pour former un sel d'alcoylthio -imidazolinium de formule générale XV

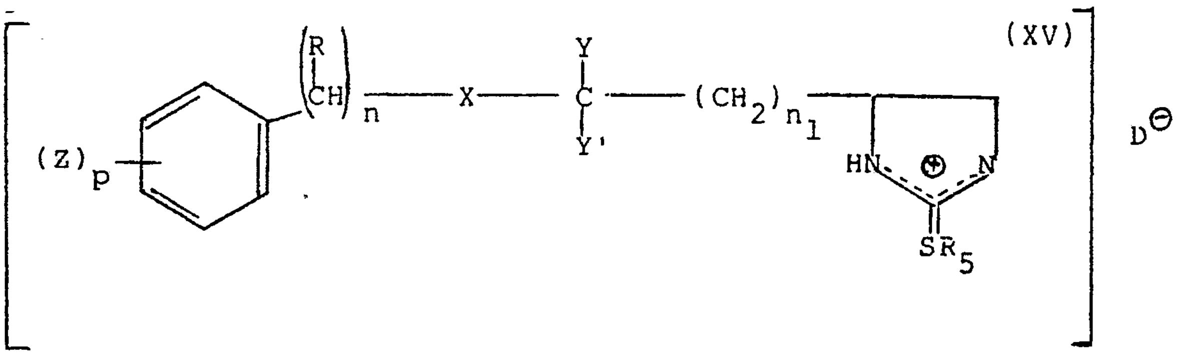

(XV)

dans laquelle les substituants gardent les significations antérieures et $R_5$ représente un radical alcoyle inférieur et D représente un anion minéral ou organique qui salifie la base et condense ce dernier avec une amine de formule générale XVI

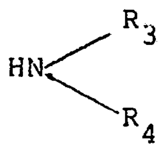

(XVI)

dans laquelle $R_3$ et $R_4$ sont définis comme précédemment sans qu'ils puissent représenter simultanément de l'hydrogène pour obtenir un composé de formule générale $I_A$

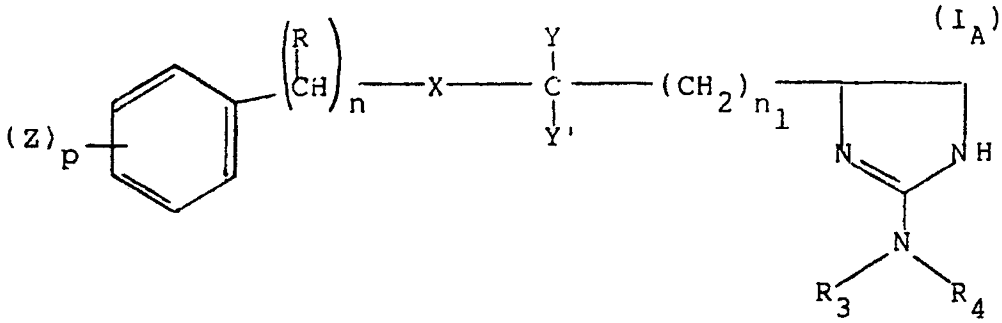

($I_A$)

dans laquelle $R_3$ représente de l'hydrogène
un radical alcoyle inférieur, alcenyle inférieur, aralcoyle inférieur ou (hétéroaryle) alcoyle inférieur, aryle, alcoyloxy carbonyle, acyle dérivé d'un acide organique carboxylique ayant de 1 à 10 atomes de carbone
$R_4$ représente
un radical alcoyle inférieur, alcenyle inférieur, aralcoyle inférieur ou (hétéroaryle) alcoyle inférieur, aryle, alcoyloxy carbonyle, acyle dérivé d'un acide organique carboxylique ayant de 1 à 10 atomes de carbone
ou bien $R_3$ et $R_4$ forment avec l'atome d'azote auxquels ils sont liés, la chaîne alcoylène d'une structure hétérocyclique azotée, éventuellement interrompue par un ou deux autres hétéroatomes.

Pour obtenir les dérivés 2-acylaminés il est possible également de faire réagir un dérivé fonctionnel d'acide alcoyl- aryl ou aralcoyl carboxylique avec un 4 - 5 dihydro [1H] imidazole, de formule générale XVII, selon les modalités de la réaction de Schotten-Baumann

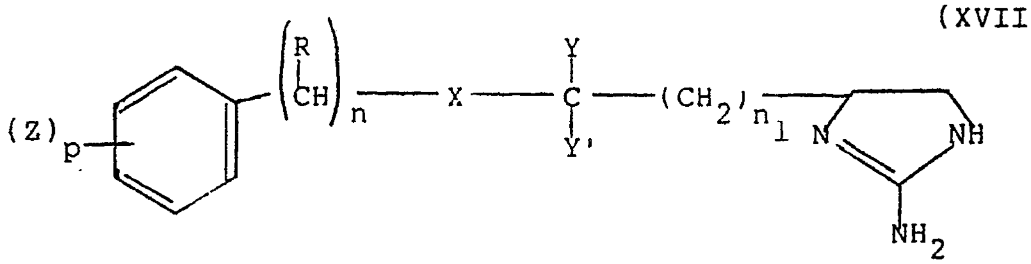

(XVII)

dans laquelle la définition des substituants Z, p, R, n, X, Y, Y' et $n_1$ demeure inchangée

Les composés de formule générale $I_A$ pour lesquels A représente un groupe NH, et le trait pointillé une double liaison, peuvent être obtenus selon un procédé qui consiste à soumettre un 1-azido 3- aryloxy 2-propanol de formule générale V

(V)

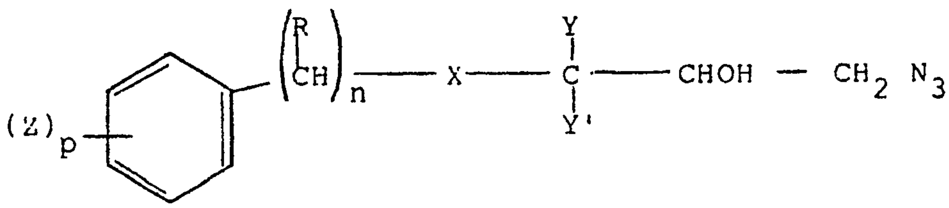

dans laquelle Z, p, R, Y, Y', R et n ont les significations fournies antérieurement
et X représente de l'oxygène ou du soufre
ou un radical N-$R_1$ ($R_1$ étant défini comme précédemment) à l'action d'un oxydant métallique en milieu acide pour obtenir une azido cetone de formule générale XVIII

(XVIII)

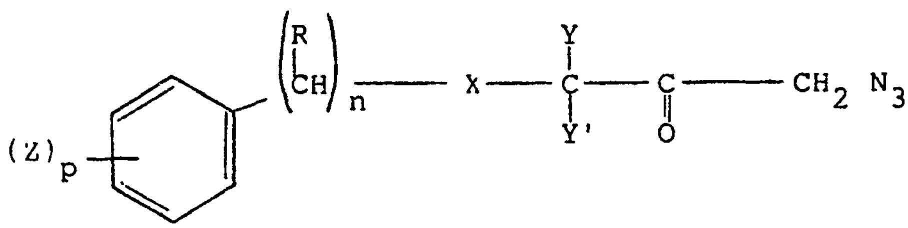

dans laquelle les substituants Z, p, R, Y, Y', X sont définis comme ci-dessus
que l'on réduit par hydrogénation catalytique en amino cétone de formule générale XIX

(XIX)

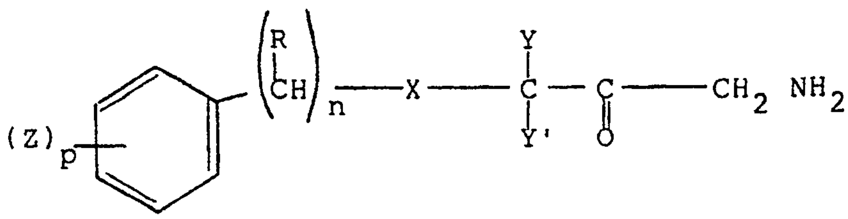

dans laquelle la définition des substituants Z, p, R, n, X, Y et Y' demeure inchangée
et condense cette dernière avec la cyanamide en présence d'une base alcaline pour obtenir un dérivé imidazolique de formule générale $I_A$

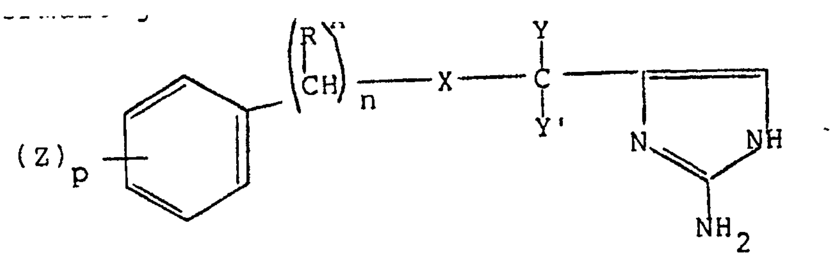

($I_A$)

dans laquelle les substituants Z, p, R, n, X, Y et Y' sont définis comme précédemment

que l'on peut salifier par addition d'un acide minéral ou organique
ou acyler par action d'un dérivé fonctionnel d'acide organique carboxylique
ou dédoubler en ses isomeres optiques.par action d'un acide optiquement actif

ou alcoyler par action d'une alcoyl cétone ou d'un aldehyde alcoylique en présence d'un agent réducteur.

Selon des modes d'exécution actuellement préférés, les procédés selon l'invention peuvent encore être définis comme suit :

1. La condensation de l'épihalohydrine de formule générale II avec le dérivé aromatique III est effectuée dans un solvant polaire inerte comme par exemple l'acétonitrile en présence d'un accepteur d'hydracide, comme par exemple le carbonate de sodium ou le carbonate de potassium.

2. L'ouverture du cycle oxirane par l'azidure de métal alcalin s'effectue en milieu aqueux comme par exemple un mélange eau-acétone ou eau-acetonitrile ou eau-dimethyl formamide.

3. Le dérivé fonctionnel d'acide sulfonique est de préférence un halogènure d'acide alcoyl-sulfonique, comme par exemple le chlorure de methane sulfonyle, le chlorure d'ethane sulfonyle. ou le chlorure d'acide trifluoromethyl sulfonyle, ou un halogènure d'acide arylsulfonyle comme par exemple le chlorure de benzene sulfonyle, le chlorure de p.toluène sulfonyle, ou chlorure d'acide naphtylsulfonyle. La condensation est effectuée en présence d'une base tertiaire comme la triethylamine, la pyridine ou la collidine.

4. La conversion du sulfonate en azide est effectuée en solvant polaire aprotique comme par exemple dans le dimethyl acetamide le dimethyl formamide, l'acetonitrile ou l'hexamethyl phosphorotriamide.

5. L'hydrogènation du dérivé azido ou di-azido est effectuée en présence d'un catalyseur comme le palladium ou le platine sur un support inerte comme le charbon, le sulfate de baryum ou le carbonate de strontium.

6. La condensation du composé aromatique III avec un halogènure d'allyle est effectuée dans un solvant polaire comme l'acetonitrile en présence d'un agent basique comme le carbonate de sodium ou le carbonate de potassium.

7. La bromation du dérivé alcénylé X est effectuée par le brome en solution dans un solvant inerte comme par exemple le tetrachlorure de carbone.

8. La conversion du dérivé dibromé en dérivé di-azido est effectuée par chauffage du dérivé bromé avec un azidure de métal alcalin dans un solvant polaire, comme le dimethyl formamid ou le dimethyl sulfoxyde.

9. La cyclisation du dérivé diaminé IX par le bromure de cyanogène est effectuée dans un solvant inerte comme un carbure aromatique à une température inférieure à 30°.

10. La cyclisation du dérivé diaminé IX par un halogènure de S-methyl Isothiouronium est réalisé par chauffage dans un solvant inerte à haut point d'ébullition, comme l'isopropanol, le propanol, la collidine ou le xylène.

11. La synthèse des composés de formule générale I peut également être effectuée au départ d'une matière première déjà dédoublée provenant d'un produit naturel que l'on dégrade comme par exemple le d-mannitol ou l'acide l-ascorbique.

De même il est possible de dédoubler les epoxydes de formule générale IV en leurs isomères géométriques et de poursuivre la synthèse pour obtenir le diastereo isomère correspondant.

L'agent de dédoublement est de préférence un acide optiquement actif comme l'acide d-tartrique, l'acide NN-diéthyl- d-tartramique, l'acide d-camphorique, l'acide l-cetogulonique, l'acide abiétique, l'acide pimarique ou l'acide d-camphosulfonique.

On peut également utiliser des moyens physiques comme par exemple la chromatographie en phase liquide à haute performance (HPLC) ou la chromatographie sur colonne chargée d'un agent absorbant optiquement actif.

On peut aussi recourir à un produit enzymatique comme l'amylase ou une hydrolase, après avoir estérifié une fonction alcool

On peut également estérifier une fonction alcool par un acide optiquement actif comme l'acide d-camphorique, séparer les ester épimériques, puis saponifier les esters optiquement-actifs.

L'invention a également pour objet des compositions pharmaceutiques contenant à titre de principe actif au moins un composé de formule générale I, ou un de ses sels d'addition avec un acide minéral ou organique en association ou en mélange avec un excipient ou un véhicule inerte non-toxique pharmaceutiquemen acceptable.

En outre, les compositions pharmaceutiques selon l'invention peuvent renfermer un autre principe actif d'action synergique ou complémentaire.

D'une manière préférée, l'excipient ou le véhicule est celui qui convient pour l'administration par voie parentérale, buccale, rectale, sublinguale, percutanée ou permuqueuse.

Parmi les formes pharmaceutiques appropriées, on citera plus particulièrement les comprimés nus ou enrobés, les dragées, les pilules, les gélules, les cachets, les capsules molles, les solutés injectables ou

buvables, les suspensions buvables, les sirops, les suppositoires, les solutions pour usage percutané ou les comprimés sublinguaux.

Les excipients ou véhicules sont par exemple les amidons, les celluloses ou les dérivés chimiques de la cellulose, comme l'ethyl cellulose ou l'hydroxypropyl cellulose, le carboxy methy amidon ou ses sels de métaux alcalins, le phosphate de magnesium le carbonate de calcium, le talc, ou le stearate de magnesium, les solutions aqueuses, salines ou non, les sirops de sucre ou de gomme, le beurre de cacao, les polyethylène glycols, les stéarates de polyethylène glycol.

Les composés de formule générale I et leurs sels se distinguent par des propriétés pharmacologiques intéressantes. Ils manifestent notamment des propriétés anti-dépressives et des propriétés cardiovasculaires dues a une action de type adrenergique. En particulier, les composés de la formule générale I ou leurs sels manifestent des effets inotropes et/ou chronotropes positifs. En outre, certains composés possedent des actions antimicrobiennes et/ou antiparasitaires et/ou antifongiques.

Ces composés peuvent trouver un emploi en médecine humaine comme médicament de la défaillance cardiovasculaire aigue et chronique, en particulier dans les états de choc, ou comme médicament psychotrope utile dans le traitement des états dépressifs endogènes ou réactionnels.

Ils sont utilisés à des doses qui peuvent varier selon l'âge, le poids du sujet, la voie d'administration et l'indication thérapeutique de 1 mg à 500 mg par prise unitaire et de 5 mg à 1000 mg par jour.

Les exemples suivants illustrent l'invention. Ils ne la limitent en aucune façon.

## EXEMPLE I

4-(0-chlorophenyloxymethyl) 2-amino 4,5 dihydro [1 H] Imidazole et son bromhydrate

### Stade A : (Orthochlorophenyloxy-1 époxy-2,3-propane)

A une solution de 62,2 ml d'orthochlorophénol dans 500 ml d'acétonitrile sec sont ajoutés 248,4 g de carbonate de potassium et 0,5 d'iodure de potassium. Après 1/2 Heure d'agitation à température ambiante, on ajoute, goutte à goutte, 140 ml d'épichlorhydrine.

Le mélange est chauffé au reflux pendant 15 heures. Après traitement habituel, on recueille par distillation 60,1 g de liquide incolore passant à 85 °C sous $2.10^{-2}$ mmHg.

### Stade B : 1-(orthochlorophenyloxy) 3-azido-propanol-2

A une solution de 51,7 g d'époxyde dans 300 ml d'acétonitrile, on ajoute 27,3 g d'azoture de sodium dissout dans 15 ml d'eau. Le mélange est porté au reflux durant 4 heures. Après traitement habituel, on obtient 61,17 g d'un liquide jaune limpide. Rendement : 96,4 % - Rf = 0,43 sur plaque de silice, en utilisant le mélange toluène 9/THF 1 comme éluant.

### Stade C : Tosylate de 1-(chlorophenyloxy) 3-azido propanol - 2

A une solution de 61,17 g d'azide dans 300 ml de pyridine mainten à -10 °C, on ajoute 66,7 g de chlorure de tosyle dans 300 ml de pyridine durant 1 heure en maintenant la température entre -5 °C et -2 °C.

Le mélange est laissé 48 heures à 4 °C puis versé sur un mélange glace/eau. Le précipité obtenu est repris au toluène et traité de manière habituelle. On obtient 90,1 g d'un solide jaune pâle. Rendement : 87,4 % - Rf = 0,63 sur plaque de silice avec le système THF l/toluène 9 comme éluant.

### Stade D : 1-(orthochlorophenyloxy)2,3-diazido -propane.

A une solution de 9,3g de tosylate dans 30 ml de DMF on ajoute 3,9 g d'azoture de sodium. L'agitation est maintenue 1/2 heure à température ambiante puis 4 heures à 90 °C. La solution résultante est versée dans l'eau et extraite à l'éther. Après traitement habituel on recueille 6,06 g d'un liquide légèrement orangé.

Rendement : 100% -Rf = 0,52 sur plaque de silice en utilisant le système éther de pétrole l/éther éthylique 1.

Stade E : 1-(orthochlorophenyloxy) 2,3-diamino-propane

Dans une fiole étanche on introduit 49,77 g de diazide dans 250 ml d'éthanol et 10 g de charbon palladié à 5%. Après avoir purgé le système à l'azote, un courant d'hydrogène est maintenu ainsi qu'une agitation vigoureuse. L'évolution de la réaction est suivie par CCM. Après filtration du charbon palladié, on recueille 40,13 g de liquide marron. La purification s'effectue par traitement acido-basique (dichlorhydrate). On obtient 28 g de base libre de 1-(0-chlorophenyloxyméthyl) 2,3-diamino propane
Rendement : 70.8%.

Stade F : 4-(orthochlorophenyloxy-méthyl) 2-amino 4,5-dihydro [1H] imidazole et son bromhydrate

A une solution de 11,32 g de 1-(0-chlorophenyloxyméthyl) 2,3-diaminopropane dans 160 ml de toluène, on ajoute, goutte à goutte, une solution de bromure de cyanogène (6,46 g) dans 60 ml de toluène. La température doit être inférieure à 30° C. L'agitation est maintenue 5 heures à température ambiante. Après filtration du solide obtenu et traitement habituel, on recueille 8,4 g d'une poudre blanche fondant à 132° C.
Rendement : 49 %.

EXEMPLE II

4-(phénoxy-méthyl) 2-phénylamino-4,5 dihydro [1H] imidazole et son fumarate.

7g de 1-phénoxy - 2,3-diamino propane (dichlorhydrate), 8,55 g d'iodhydrate de S-méthyl-phénylisothiouronium et 8,8 g de triéthyl-amine dans 50 ml de propanol sont mis à chauffer à 140° C dans un autoclave durant 12 heures. Après traitement habituel, on obtient une huile très colorée qui est purifiée par chromatographie sur colonne d'alumine avec le système $CH_2Cl_2$ 95-MeOH5 . Le fumarate est obtenu par traitement à l'acide fumarique dans l'éthanol et purifié par relargage éthanol/éther. On obtient ainsi 2,25 g d'une poudre blanche fondant à 174° C.
Rendement : 23 %.

EXEMPLE III

4-benzyloxy 2-ethylamino 4,5-dihydro [1H] imidazole

Stade A : 3-benzyloxy-propène-1

A un mélange de 432 g d'alcool benzylique et 31,75 g de poudre de Cu chauffé à 40° C, on ajoute, goutte à goutte, 153 g de chlorure d'allyle redistillé. L'ensemble est maintenu sous reflux 4 heures en ajoutant de temps en temps du carbonate de sodium. Après filtration des produits minéraux et traitement habituel, le liquide résultant est chromatographié sur colonne de silice avec le système éther de pétrole 5/éther éthylique 95 On obtient 66 g du produit attendu.
Rendement : 44,6 %.

Stade B : 1-benzyloxy-2,3-dibromo-propane

A une solution de 162g5 de 3-benzyloxy propène-1 dans 1000 ml de tétrachlorure de carbone maintenu à 0° , on ajoute goutte à goutte 56,5 ml de brome en solution dans 100 ml de tétrachlorure de carbone. Après traitement habituel, on obtient un produit huileux jaune qui est utilisé pour la suite de la synthèse

sans autre purification.
Le rendement en produit brut est de 98 %.

Stade C : 1-benzyloxy 2,3-diazidopropane

On dissout 50 g de dérivé dibromé obtenu au stade B dans 300 ml de dimethylformamide et on y ajoute 26g4 d'azoture de sodium. On maintient sous agitation pendant 12 heures tout en chauffant à 80° C. On ajoute ensuite de l'eau et on traite le résidu de la manière habituelle. On recueille 31g45 d'un produit huileux coloré en jaune.
Le rendement de la réaction est de 83,5 %.

Stade D :

En opérant comme à l'exemple I Stade E, on obtient le 1-benzyloxy 2,3-diamino propane.

Stade E : 4-benzyloxy 2-ethylamino 4,5-dihydro [IH] imidazole et son chlorhydrate.

7g5 de 1-benzyloxy 2,3-diaminopropane sous forme de dichlorhydrate, 8g1 d'iodhydrate de S-methyl ethylisothiouronium et 8g8 de triethylamine en solution dans 50 ml de propanol sont chauffés à 140° C à l'autoclave pendant 12 heures. Après retour à la température ordinaire, on amène le mélange à sec. Le résidu huileux est purifié par chromatographie sur colonne de silice. On le redissout dans 25 ml d'ether que l'on sature par un courant de gaz chlorhydrique. Le chlorhydrate précipite progressivement On laisse reposer une nuit en glacière, puis sépare les cristaux que l'on essore puis rince par un peu d'ether. Après séchage en étuve, on recueille 4g45 de chlorhydrate de 4-benzyloxy 2-ethy lamino 4,5-dihydro [1H] imidazole.

EXEMPLE IV

4-(parachlorobenzyloxy-méthyl) 2-(N-carbethoxy-amino) 4,5-dihydro [1H] imidazole

A une solution de 10 g de sulfate de S-méthylisothiourée dans 8 ml d'eau, on ajoute 3,6 ml de chloroformiate d'éthyle. Le mélange est refroidi à + 5° C et 5,5 ml de soude à 20 % sont ajoutés. L'agitation est maintenue 5 heures à température ambiante. Après traitement habituel, le produit résultant sous forme d'huile jaune pâle est ajouté à 3,5 g de chlorhydrate de 2,3-diamino-(parachlorophénoxy-méthyl) - propane et à 2,14 g d'hydrogénocarbonate de sodium dans 100 ml de méthanol. Le méthanol est distillé puis remplacé par 150 ml de n-propanol que l'on porte à reflux pendant 10 Heures. Le traitement habituel donne 2,17 g d'un solide fondant à 191° C (chlorhydrate)
Rendement : 54 %.

Tous les composés décrits ont des spectres infra-rouge et de résonnance magnétique nucléaire en accord avec leurs structures.

EXEMPLE V

4-(benzodioxanyl-2) 2-amino amidazolines et leurs bromhydrates ainsi que leurs isomères threo et erythro

Stade A :

Au départ du 2-(benzodioxanyl-2) 2-hydroxy 1-bromoethane et de l'azidure de sodium en solution dans le dimethyl formamide à une température de l'ordre de 80° C on obtient le 2-(benzodioxanyl-2) 2-hydroxy 1-azidoethane.

Stade B :

En opérant selon le mode opératoire de l'exemple I, stade C, au départ du 2-(benzodioxanyl-2) 2-hydroxy 1-azidoethane et du chlorure de p.toluène sulfonyle on obtient le 2-(benzodioxanyl-2) 2-p.toluène sulfonyloxy 1-azidoethane.

Stade C :

En opérant selon le mode opératoire de l'exemple I, stade D, on transforme le 2-(benzodioxanyl-2) 2-(p.toluène sulfonyloxy) 1-azidoethane en 2-(benzodioxanyl-2) 1, 2-diazidoethane qui se prête à la séparation des isomères erythro et threo du mélange par chromatographie sur colonne de silice.

Stade D :

En opérant comme à l'exemple I stade E, au départ du 2-(benzodioxanyl-2) 1,2-diazidoethane - mélange d'isomères erythro et threo ou sous forme dédoublée - on obtient le 2-(benzodioxanyl-2) 1,2-diaminoethane correspondant.

Stade E :

En opérant comme à l'exemple I stade F, au départ du 2-(benzodioxanyl-2) 1,2-diaminoethane on obtient par action du bromure de cyanogène une 2-amino 4-(benzodioxanyl-2) imidazoline que l'on purifie par conversion en bromhydrate.

| P.M (base) | F°C (bromhydrate) | Analyse Elémentaire Calculé | | | Trouvé | | |
|---|---|---|---|---|---|---|---|
| | | C | H | N | C | H | N |
| 219,25 | 225 | 44,01 | 4,7 | 13,99 | 44,06 | 4,81 | 13,81 |
| 219,25 | 180 | 44,01 | 4,7 | 13,99 | 43,89 | 4,82 | 13,72 |
| | (isomères threo et erythro) | | | | | | |

EXEMPLE VI

En opérant selon le mode opératoire de l'exemple III, les composés suivant ont été préparés :

a) 4-(α-naphtyloxymethyl) 2-amino 4,5-dihydro [1H] imidazol sous forme de bromhydrate

F = 175°C PM = 241.295 (base)

Analyse :

| Calculé | | | Trouvé | | |
|---|---|---|---|---|---|
| C | H | N% | C | H | N% |
| 52,19 | 5,01 | 13,04 | 51,98 | 4,92 | 12,90 |

b) 4-(β-naphtyloxymethyl) 2-amino 4,5-dihydro [1H] imidazole sous forme de bromhydrate

F = 194°C

Analyse :

| | C | H | N% |
|---|---|---|---|
| Calculé | 52,19 | 5,01 | 13,04 |
| Trouvé | 52,32 | 5,11 | 12,98 |

c) 4-(2-méthyl 4-bromophenoxy) 2-amino 4,5-dihydro [1H] imidazole sous forme de bromhydrate

F = 190°C

Analyse :

| | C | H | N% |
|---|---|---|---|
| Calculé | 36,19 | 4,14 | 11,51 |
| Trouvé | 36,34 | 4,39 | 11,46 |

d) 4-(2-méthylphénoxy) 2-amino 4,5-dihydro [1H] imidazole sous forme de bromhydrate

F = 125°C

Analyse :

| | C | H | N% |
|---|---|---|---|
| Calculé | 46,17 | 5,64 | 14,68 |
| Trouvé | 46,05 | 5,75 | 14,53 |

e) 4-(3,4-méthylène dioxyphenoxy) 2-amino 4,5-dihydro [1H] imidazole sous forme de bromhydrate

F = 150°C

Analyse :

| | C | H | N% |
|---|---|---|---|
| Calculé | 41,78 | 4,46 | 13,29 |
| Trouvé | 41,96 | 4,63 | 13,06 |

f) 4-(phénoxy) 2-amino 4,5-dihydro [1H] imidazole sous forme de bromhydrate

F = 145°C

Analyse :

| | C | H | N% |
|---|---|---|---|
| Calculé | 44,13 | 5,19 | 15,44 |
| Trouvé | 44,38 | 5,30 | 15,20 |

g) 4-(2-méthoxyphenoxy) 2-amino 4,5-dihydro [1H] imidazole sous forme de bromhydrate

F = 128°C

Analyse :

| | C | H | N% |
|---|---|---|---|
| Calculé | 43,72 | 5,34 | 13,91 |
| Trouvé | 43,88 | 5,50 | 13,84 |

h) les imidazolines suivantes ont encore été préparées :

EXEMPLE VII 4-(2, 2-diphenylethyl-1) 2-amino [1H] 4, 5-dihydro imidazole.

Il est préparé à partir du 4, 4- diphenylbutène-1, décrit dans le brevet Français 2.313.022, par l'intermédiaire du dérivé dibromé, du dérivé diazido puis réduction en dérivé diaminé puis cyclisation par action du bromure de cyanogène. Le produit isolé sous forme de bromhydrate fond à 175° C.

Analyse $C_{17} H_{19} N_3$, BrH = 265,31

| | C | H | N% |
|---|---|---|---|
| Calculé | 58,95 | 5,82 | 12,13 |
| Trouvé | 59,11 | 5,95 | 12,10 |

EXEMPLE VIII

4-[(N-phenyl N-p. toluène sulfonyl amino) methyl] 2-amino [1H] 4, 5-dihydro imidazole.

Ce composé est préparé à partir de la N-phenyl N- allyl p. toluène sulfonamide (décrit dans Organ. Khim. 1(1965) 918) par l'intermédiaire du dibromé, du dérivé diazido et réduction en dérivé diaminé, puis cyclisation par action du bromure de cyanogène. Le produit est isolé sous forme de bromhydrate cristallisé avec une 1/2 molécule d'eau.

F = 126 - 127°C.

Analyse $C_{17}H_{19}N_4SO_2$, BrH, 1/2 $OH_2$ = 344,43

| | C | H | N% |
|---|---|---|---|
| Calculé | 47,01 | 5,10 | 12,89 |
| Trouvé | 47,32 | 6,07 | 12,84 |
| | 47,43 | 5,98 | 12,82 |

EXEMPLE IX

[ Dibenzo (a,d) [5H] 10, 11-dihydrocycloheptenyl-5] 4-methyl 2-amino [1H] 4,5 -dihydro imidazole.

Ce composé est obtenu sous forme de bromhydrate au départ du 5-allyl [dibenzo (a, d) [5H] 10, 11-dihydro cycloheptene]-préparé selon le procédé décrit dans le brevet Belge 633.597 - par transformation en dérivé dibromé, conversion en dérivé diazido réduction en diamine, puis cyclisation par le bromure de cyanogène.
Le produit cristallise avec 1/3 de molécule d'eau.
F> 55° C puis décomposition.

Analyse $C_{19}H_{23}N_1$, BrH, 1/3 $H_2O$ = 291,41

| | C | H | N% |
|---|---|---|---|
| Calculé | 60,32 | 5,95 | 11,10 |
| Trouvé | 60,48 | 5,96 | 11,22 |

EXEMPLE X

4- [ (2-nitrophenoxy) phenylmethyl] [1H] 4, 5-dihydro imidazole.

Ce composé est préparé à partir du (2-nitrophenoxy-3-phenyl - 2-hydroxypropionate d'éthyle - obtenu selon la méthode décrite dans J. of Heterocyclic Chem. 20 (1983) 259 - par hydrogénation en diol, conversion de celui-ci en di-mesylate, puis en diazide et réduction en diamine que l'on cyclise par le bromure de cyanogène.
Le produit isolé sous forme de bromhydrate fond à 237° C.

EXEMPLE XI

4- phenoxymethyl 1, 4, 5, 6-tetrahydropyrimidine. a) 4-phenoxy butane 1, 3-diol.

On dissout 28 g (0,12 mol) de 4-phenoxy 3-oxobutanoate d'ethyle dans 200 ml d'ether ethylique et on coule cette solution dans une suspension de 13g6 d'aluminohydrure de lithium dans 500 ml d'ether

refroidie à 0°. L'agitation est maintenue 2 heures à température ordinaire. On hydrolyse l'excès de réactif par addition ménagée d'une solution aqueuse de sulfate de sodium. On filtre puis évapore à sec le filtrat. Le résidu est repris dans l'ether de pétrole où il cristallise. On obtient 20 g de produit, soit un rendement de 92 %. (PF = 52 - 54°C).

Spectre RMN dans $CDCl_3$

$\tau$ entre 6,7 et 7,3
entre 9,15 et 4

Le produit est ensuite transformé en ditosylate, puis en diazide, réduit en dérivé diaminé et cyclisé en tetrahydropyrimidine.

EXEMPLE XII

2-amine - 4 benzyl [1H] imidazole

F du fumarate = 200°C

Analyse (fumarate) $C_{12}H_{13}N_3O_2$ = 231,257

| | C | H | N% |
|---|---|---|---|
| Calculé | 62,33 | 5,67 | 18,17 |
| Trouvé | 62,10 | 5,62 | 18,00 |

EXEMPLE XIII

4-(o. chlorophenoxy methyl) 2-N-morpholino 4, 5 - dihydro [1H] imidazole

Stade A.

4-(o. chlorophenoxy methyl) 2-thio 4, 5-dihydro [1H] imidazole 0,2 mole de 3-orthochlorophénoxy -1,2-diaminopropane est dissout dans 400cm3 d'éthanol à 80%, on ajoute goutte à goutte et sous argon 12cm3 de sulfure de carbone (0,2 mole). Le mélange est chauffé à reflux durant 1 heure. Après refroidissement, on ajoute goutte à goutte 0,35 cm3 d'HCl concentré. Le mélange est laissé 6 heures à reflux. Après refroidissement on ajoute 1 volume d'eau; on recueille 25,4 g d'un précipité jaune.
Point de fusion = 148°C.
Recristallisation dans du chlorure de méthylène.
Rendement = 52%.

Stade B.

Iodhydrate de 4-(C.chlorophenoxymethyl)2-(methyl thio) imidazolidinium.

A 0,08 Mole de composé obtenu au Stade A, on ajoute 6,25cm3 (14,2g ; 0,1 mole) d'iodure de méthyle. On chauffe 1 heure à reflux. Le mélange est concentré sous vide. Le solide obtenu est lavé à l'éther, après

sèchage on obtient 29,8g de solide jaune fondant à 174° C.
Après recristallisation dans l'isopropanol.
Le point de fusion s'élève à 175° C.
Rendement = 94%.

Stade C.

4-(orthochlorophénoxyméthyl) 2-N-morpholino - 4,8 dihydro [1H] imidazole.

A un équivalent (6g ; 0,0155 mole) de l'iodure obtenu au Stade B, on ajoute 2,7 g (2eq.) de morpholine dans 100 cm3 d'isopropanol. Le mélange est chauffé 24 heures à reflux. Après évaporation du solvant, reprendre à l'eau et laver à l'éther. On ajoute alors de la soude à 10% jusqu'à neutralité, et on extrait au chlorure de méthylène ; la phase organique est lavée à l'eau, séchée et évaporée. La purification s'effectue en précipitant le fumarate dans l'éthanol. Le sel est recristallisé dans le méthanol.
Point de fusion = 184° C.
Rendement = 81 %
Les autres dérivés N-substitués sont obtenus d'une manière analogue en faisant réagir le dérivé thio-méthylé dans un solvant comme l'isopropanol à reflux, ou entre 80° C et 140° C à l'autoclave, avec les amines convenablement choisies.

EXEMPLE XIV

4-(o.chlorophenoxy methyl) 2-benzoylamino 4, 5-dihydro [1H] imidazole.

A 6g (0,02 Mole, leq.) de 4-(orthochlorophénoxy-méthyl) -2 amino 4,5- dihydro [1H] imidazole dissouts dans 200 cm3 d'eau, on ajoute de la soude N jusqu'à pH = 10,5. Sous bonne agitation on ajoute une solution de chlorure d'acide benzoïque diluée dans du toluène (3,5 ml de chlorure d'acide dans 20 ml de toluène). L'introduction goutte à goutte dure environ 2 Heures en ajoutant parallèlement de la soude N de façon à maintenir le pH entre 7 et 9. Un solide blanc apparaît. L'agitation est maintenue 5 heures après l'introduction. Le solide est filtré, lavé à l'eau puis au toluène puis à l'acétate d'éthyle. Le solide blanc obtenu est recristallisé dans l'éthanol à 95°.
Le benzamide fond à 213° C.
Rendement = 49,6 %.

EXEMPLE XV.

4-benzyl 2-aminothiazole.

A 10 g de 1-chloro-3- phényl-2 propanone (0,058 mole) on ajoute 8,9g (0,117 mole) de thiourée dans 100 cm3 d'éthanol. Le mélange est mis sous reflux 2 Heures. Après refroidissement le tout est concentré sous vide. Le résidu est repris à l'ammoniaque diluée et extrait de l'éther. Après traitement le solide obtenu est purifié par formation de son chlorhydrate.
PF = 127° C.
Rendement = 79 %.

Analyse $C_{10}H_{11}ClN_2S$ = 226,72

| | C | H | N% |
|---|---|---|---|
| Calculé | 52,98 | 4,89 | 12,36 |
| Trouvé | 52,58 | 4,96 | 12,30 |

De la même façon on a préparé :

- le 4-(phenoxyméthyl) 2-aminothiazole dont le méthane sulfonate fond à 153° C.

Analyse $C_{11}H_{14}N_2O_4S_2$ = 302,37

| | C | H | N% |
|---|---|---|---|
| Calculé | 43,69 | 4,67 | 9,26 |
| Trouvé | 43,86 | 4,78 | 9,20 |

- le 4-(phenoxyéthyl) 2-aminothiazole dont le chlorhydrate fond à 174° C.

Analyse $C_{11}H_{13}ClN_2OS$ = 256,75

| | C | H | N% |
|---|---|---|---|
| Calculé | 51,46 | 5,10 | 10,91 |
| Trouvé | 51,36 | 5,21 | 10,98 |

Tableaux. Les composés selon l'invention figurant dans les tableaux I et II ci-après annexés ont encore été préparés.

EXEMPLE XVI

Etude pharmacologique des composés selon l'invention. Les composés de formule générale I se distinguent par un profil pharmacologique intéressant :

1) Ils possèdent, en particulier, des propriétés antidépressives, notamment mises en évidence aux test d'hypothermie et de ptosis à la réserpine, d'hypothermie à l'apomorphine (actifs de 50 à 200 mg/kg par voie orale) et de toxicité à la yohimbine chez la souris (actifs de 25 à 100 mg/kg par voie orale), et au test de "désespoir" chez la souris (actifs de 20 à 40 mg/kg par voie intrapéritonéale) ; ces propriétés antidépressives ne sont pas assorties d'effets anticholinergiques (inactivité vis-à-vis des effets centraux et périphériques de l'oxotrémorine).

2) Dans le domaine cardiovasculaire chez le chien anesthésié, ils ont des propriétés hypertensives, inotropes et chronotropes positives de longue durée, à des doses allant de 0,1 à 1 mg/kg par voie veineuse, sans effet vasoconstricteur apparent. Ces propriétés ont pour conséquence d'augmenter le débit cardiaque et la perfusion tissulaire. Dans le domaine du choc cardiovasculaire expérimental provoqué, par exemple, par injection d'endotoxine ou de PAF (platelet activating factor), les composés décrits, aux doses déjà citées et par la même voie d'administration, s'opposent au collapsus cardiovasculaire qui en découle.

**IMIDAZOLINES**

| (Z) | Y | Y' | $R_3$ | $R_4$ | n | m | p | F°C (sels) | Analyse Elementaire (Sels) Calculée C | H | N | Trouvée C | H | N |
|---|---|---|---|---|---|---|---|---|---|---|---|---|---|---|
| H | H | H | $C_6H_5$ | H | O | O | O | 174 (a) | 62,49 | 5,44 | 10,78 | 62,65 | 5,52 | 10,96 |
| H | H | H | 2,6-$Cl_2C_6H_3$ | H | O | O | O | 240 (a) | 52,98 | 4,30 | 9,10 | 53,11 | 4,23 | 9,29 |
| H | H | H | 2,6-$Cl_2C_6H_3$ | H | 1 | O | O | 145 (b) | 48,36 | 4,61 | 9,43 | 48,43 | 4,74 | 9,41 |
| H | H | H | H | H | 1 | O | O | 100 (c) | 46,30 | 5,66 | 14,50 | 46,17 | 5,64 | 14,68 |
| 3,4$(OCH_3)_2$ | H | H | H | H | O | O | 1 | 174 (c) | 43,59 | 5,47 | 12,51 | 43,39 | 5,46 | 12,65 |
| 3,4$(OCH_3)_2$ | H | H | 2,6-$Cl_2C_6H_3$ | H | O | O | 1 | 165 (b) | 46,44 | 4,65 | 8,66 | 46,35 | 4,71 | 8,53 |
| 4-Cl | H | H | H | H | O | O | 1 | 142 (c) | 39,06 | 4,37 | 13,56 | 39,17 | 4,27 | 13,70 |
| 4-Cl | H | H | H | COOEt | O | O | 1 | 191 | 46,93 | 4,93 | 12,43 | 46,72 | 5,13 | 12,57 |
| 2,6-$Cl_2$ | H | H | H | H | O | O | 2 | 99 (c) | 35,21 | 3,72 | 12,20 | 35,22 | 3,55 | 12,32 |
| 2-Cl | H | H | H | H | O | O | 1 | 132 (c) | 39,14 | 4,38 | 13,88 | 39,176 | 4,27 | 13,71 |
| H | H | H | H | COOEt | 1 | O | O | 121 | 60,32 | 6,96 | 15,09 | 60,63 | 6,91 | 15,15 |
| 4-Cl | H | H | 2,6-$Cl_2C_6H_3$ | H | O | O | 1 | 210 (b) | 43,61 | 3,99 | 8,94 | 43,74 | 3,89 | 9,00 |

(c) = bromhydrate (b) = sulfonate (a) = fumarate

## TABLEAU II

| Z | Y | Y' | P | R | n | X | $n_1$ | m | A | $R_3$ | $R_4$ | P.M (Base) | F°C bromhydrate | Analyse Elémentaire Calculée C | Calculée H | Calculée N | (Bromhydrate) Trouvée C | Trouvée H | Trouvée N |
|---|---|---|---|---|---|---|---|---|---|---|---|---|---|---|---|---|---|---|---|
| 2-Cl | H | H | 1 | H | O | O | 1 | O | NH | H | H | 239,70 | 135 | 41,21 | 4,72 | 13,11 | 41,21 | 4,83 | 12,62 |
| 2,3-Cl 4-MeO | H | H | 3 | H | O | O | O | O | NH | H | H | 290,15 | 208 | 35,61 | 3,80 | 11,32 | 35,73 | 3,94 | 11,37 |
| 2-Cl | H | H | 1 | H | - | O | O | O | NH | H | H (isomère R (+)) | 225,67 | 50 | 39,18 | 4,27 | 13,7 | 38,55 | 4,85 | 10,62 |
| 2-Cl | H | H | 1 | H | - | O | O | O | NH | morpholine | | 295,76 | $184^1$ | 52,50 | 5,38 | 10,20 | 52,42 | 5,39 | 10,14 |
| 2-Cl | H | H | 1 | H | - | O | O | O | NH | pipéridine | | 293,79 | $149^1$ | 55,68 | 5,90 | 10,25 | 55,53 | 5,78 | 10,18 |
| 3,4-Cl | H | H | 2 | H | - | O | O | O | NH | H | H | 260,13 | 178 | 35,2 | 3,54 | 12,32 | 34,99 | 3,76 | 12,30 |
| 2-Cl | H | H | 1 | H | - | O | O | O | NH | pipérazine | | 294,78 | $168^1$ | 50,15 | 5,16 | 10,63 | 50,05 | 5,26 | 10,55 |
| 2-Cl 3,4-diMeO | H | H | 3 | H | - | O | O | O | NH | H | H | 285,73 | 120 | 39,31 | 4,67 | 11,46 | 39,52 | 4,81 | 11,62 |
| 2-Cl | H | H | 1 | H | - | O | O | O | NH | 4-(O-méthoxyphényl)-pipérazine | | 400,90 | $171^1$ | 58,06 | 5,65 | 10,84 | 57,95 | 5,74 | 10,77 |
| 2-Cl | H | H | 1 | H | - | O | O | O | NH | H | $CH_3$ | 239,70 | $152^1$ | 50,64 | 5,10 | 11,81 | 50,65 | 5,33 | 11,77 |
| 2,4-Cl | H | H | 2 | H | - | O | O | O | NH | H | H | 261,13 | 194 | 35,22 | 3,54 | 12,32 | 35,48 | 3,68 | 12,24 |
| 2-Cl | H | H | 2 | H | - | O | O | O | NH | H | $NH_2$ | 240,69 | $142^2$ | 32,59 | 3,83 | 15,20 | 32,69 | 3,99 | 15,30 |
| 4-Br | H | H | 2 | H | - | O | O | O | NH | H | H | 270,13 | 178 | 34,21 | 3,73 | 11,96 | 34,28 | 3,87 | 11,95 |
| 2,5-Cl | H | H | 2 | H | - | O | O | O | NH | H | H | 260,13 | 94 | 35,2 | 3,54 | 12,32 | 35,25 | 3,64 | 12,20 |
| H | H | H | | H | - | S | O | O | NH | H | H | 207,29 | 202 | 41,67 | 4,90 | 14,58 | 41,70 | 4,94 | 14,57 |
| 2-Cl | H | H | 1 | H | - | O | O | O | NH | 4-(pyrimidino-2)-pipérazine | | 372,85 | $208^1$ | 54,05 | 5,15 | 17,19 | 53,88 | 5,15 | 17,05 |
| 2-Cl | H | H | 1 | H | - | O | O | O | NH | $C_2H_5$ | $C_2H_5$ | 281,78 | $173^1$ | 54,34 | 6,08 | 10,56 | 54,24 | 6,03 | 10,55 |
| (cyclohexène-2) | H | H | 1 | H | - | O | O | O | NH | H | H | 271,36 | $196^1$ | 62,00 | 6,50 | 10,84 | 61,84 | 6,66 | 10,84 |
| (cyclohexane-2) | H | H | 1 | H | - | O | O | O | NH | H | H | 273,38 | 149 | 54,24 | 6,83 | 11,86 | 54,08 | 6,76 | 11,82 |
| 4-$SCH_3$ | H | H | 1 | H | - | O | O | O | NH | H | H | 237,32 | 157 | 41,52 | 5,07 | 13,20 | 41,75 | 5,11 | 13,01 |
| 2-Cl | H | H | 1 | H | - | O | O | O | NH | H | 3,4-diméthoxyphényl-éthane | 375,85 | <$50^1$ | 56,16 | 5,33 | 8,54 | 53,99 | 5,67 | 8,52 |
| 2-Cl | H | H | 1 | H | - | O | O | O | NH | H | (4-hydroxyphényl)-2-méthyl-1-éthane | 359,85 | <$50^1$ | 58,05 | 5,51 | 8,63 | 55,32 | 5,65 | 8,64 |
| 2-Cl | H | H | 1 | H | - | O | O | O | NH | H | H °isomère S(-) | 225,67 | <50 | 39,17 | 4,27 | 13,7 | 39,16 | 4,37 | 13,58 |

1 = méthane-sulfonate
2 = fumarate

## Revendications

1. Les composés répondant à la formule générale I

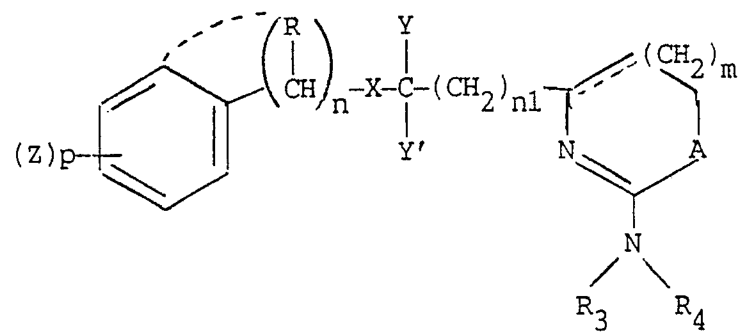

(I)

dans laquelle Z est de l'hydrogène, un atome d'halogène, un radical alcoyle inférieur, un radical alcoxy inférieur, un radical trifluorométhyle, un radical trifluoro méthoxy, cyano, nitro, carboxamido, un radical alcényle inférieur, un radical (alcoyle inférieur) thio, un groupe alcoylène dioxy, un radical cycloalcényle ou cycloalcoyle inférieur

X représente de l'oxygène, du soufre, un radical imino de formule >NH
une liaison directe ou un radical méthylène

Y représente de l'hydrogène, un radical alcoyle inférieur, un radical phényle, un hydroxyle ou un radical phénoxy

Y' représente de l'hydrogène

ou bien Y et Y' forment ensemble l'oxygène d'un groupe carbonyle

ou bien Y forme avec le radical phényle adjacent, lorsque n est égal à zéro, une structure bicyclique hétérocyclique choisie dans le groupe constitué par le tétrahydroindole, le dihydrobenzothiophène et le thiochromanne

A représente un groupe NH, ou du soufre

$R_3$ et $R_4$ semblables ou différents, représentent de l'hydrogène, un radical alcoyle inférieur, alcényle inférieur, aralcoyle inférieur dont la chaine hydrocarbonée a de 1 à 6 atomes de carbone en chaine droite ou ramifiée choisi dans le groupe constitué par le radical benzyle, phénéthyle, $\alpha$-méthylphényl éthyle, 2,6-dichlorobenzyle et 2,3,5-triméthoxybenzyle ou (hétéroaryl alcoyle) inférieur, dont la chaine hydrocarbonée a de 1 à 6 atomes de carbone choisi dans le groupe constitué par le pyridyl-2 méthyle, le furyléthyle, le pyranyléthyle et le thiényl-2 méthyle, alcoxycarbonyle, un acyle dérivé d'un acide organique carboxylique ayant de 1 à 10 atomes de carbone ou un radical amino, ou bien $R_3$ et $R_4$ forment avec l'atome d'azote auxquels ils sont liés, la chaine alcoylène d'une structure hétérocyclique azotée, éventuellement interrompue par un ou deux autres hétéro-atomes

R représente un hydrogène, un radical alcoyle inférieur, un radical phénylène relié au noyau benzénique par une chaine alcoylène ayant 1 ou 2 atomes de carbone

n est égal à zéro ou 1

$n_1$ est égal à 0, 1 ou 2

m est égal à 0, 1

et p est égal à 1, 2 ou 3 le trait pointillé symbolise une double liaison carbone-carbone éventuelle,les radicaux alcoyle inférieur et alcoxy inférieur ayant de 1 à 6 atoms de carbone, le radical alcényle inférieur ayant de 2 à 6 atomes de carbone et le radical alcoylène dioxy ayant de 1 à 4 atoms de carbone.

2. Les sels des composés selon la revendication 1 par addition avec un acide minéral ou organique

3. Les formes tautomères des composés selon l'une des revendications 1 et 2.

4. Les formes optiquement actives des composés selon l'une des revendications 1 à 3.

**5.** Les composés selon la revendication 1 répondant à la formule générale I"

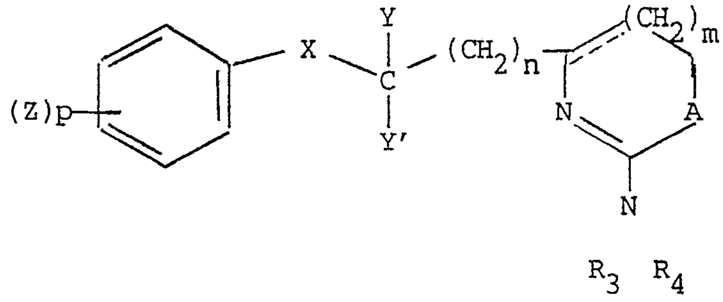

(I")

qui correspondent au cas ou n est égal à zéro, et dans laquelle X représente de l'oxygène, du soufre, un radical imino de formule >NH, un radical méthylène ou une liaison directe.

**6.** Les composés selon la revendication 1 répondant à la formule générale I'''

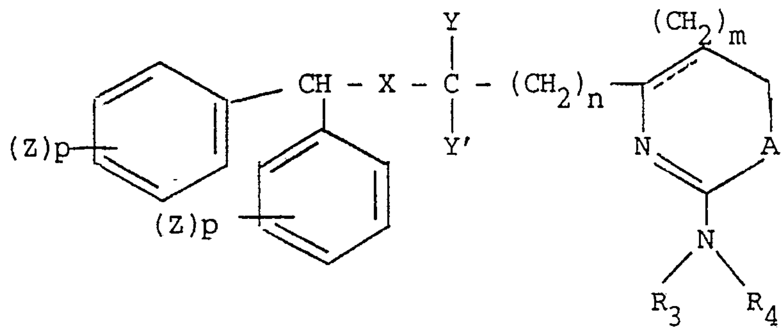

(I''')

dans laquelle la définition des substituants X, Y, Y', Z, A, $R_3$, $R_4$, $n_1$ et p demeure celle fournie à la revendication 1.

**7.** Les composés selon la revendication 1 répondant à la formule générale I""

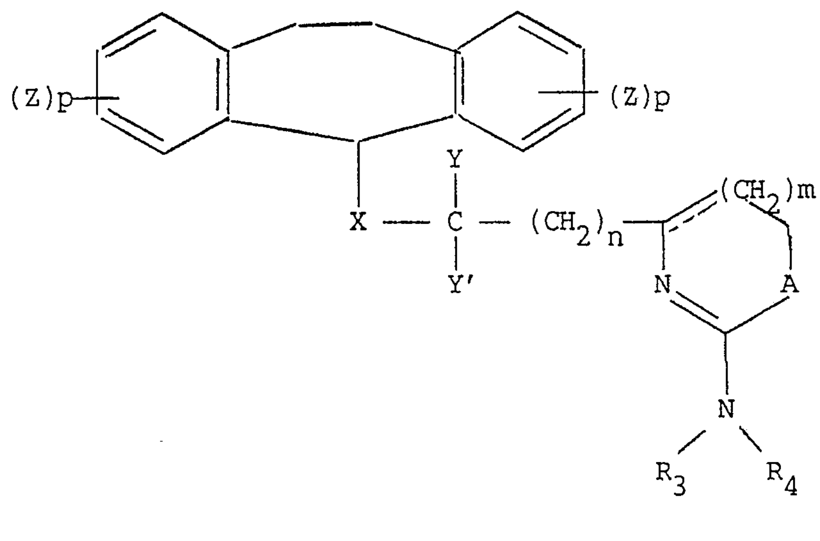

(I"")

dans laquelle la définition des substituants Z, X,Y, Y', $R_3$, $R_4$, A, m, n et p demeure celle fournie à la revendication 1.

**8.** Les imidazolines de formule générale $I_A$ selon la revendication 1.

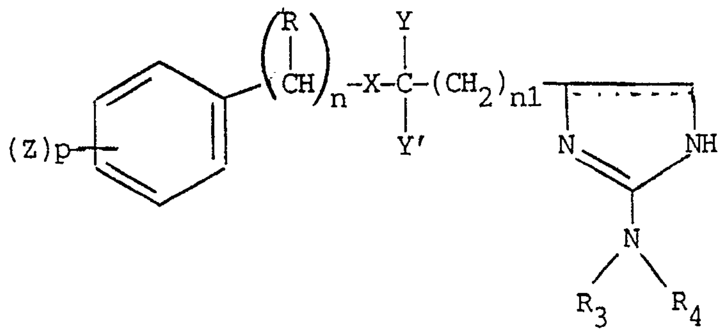

$(I_A)$

dans laquelle les substituants X, Y, Y', Z, R, $R_3$, $R_4$, n, $n_1$ et p ont les significations fournies précédemment
et m est égal a zéro

9. Les tétrahydropyrimidines de formule générale $I_B$ selon la revendication 1

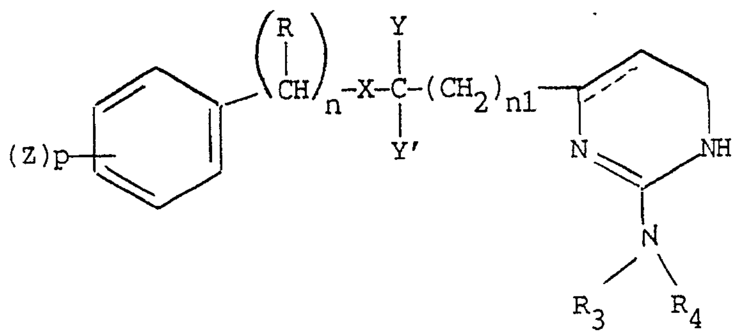

$(I_B)$

dans laquelle les substituants X, Y, Y', Z, $R_3$, $R_4$, n et p ont les définitions fournies antérieurement et m est égal à 1

10. Les 2-aminothiazoles de formule générale $I_C$ selon la revendication 1

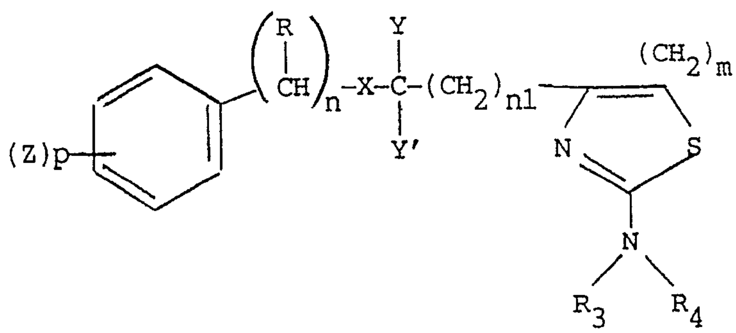

$(I_C)$

dans laquelle les substituants X, Y, Y', Z, R, $R_3$, $R_4$, n, $n_1$ et p ont les définitions fournies antérieurement a la revendication 1.

11. Un procédé d'obtention des composés de formule générale I selon la revendication 1 dans laquele m est égal à zéro, qui consiste en ce que l'on condense une épihalohydrine de formule générale II

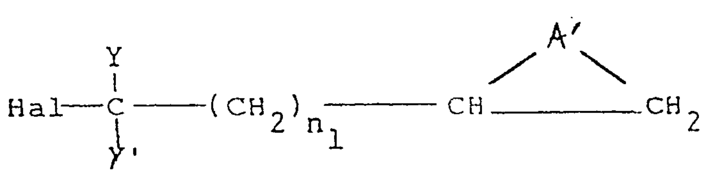

(II)

dans laquelle Hal est du chlore ou du brome A' est de l'oxygène ou du soufre
les substituants Y, Y' et $n_1$ ont les significations fournies antérieurement
et m est égal à 0
avec un dérivé aromatique de formule générale III

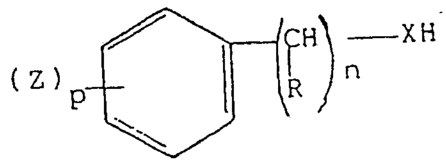

(III)

dans laquelle les substituants R, Z, n, p et X sont définis comme ci-dessus
pour obtenir un dérivé aralcoylé de formule générale IV

(IV)

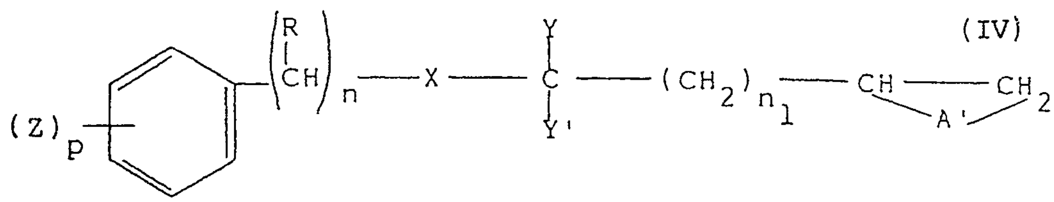

dans laquelle la définition des substituants R, A', X, Y, Y', Z, n, $n_1$ et p demeure inchangée
dont on ouvre le cycle oxirane par action d'un azidure de métal alcalin pour former un mono azide de formule générale V

(V)

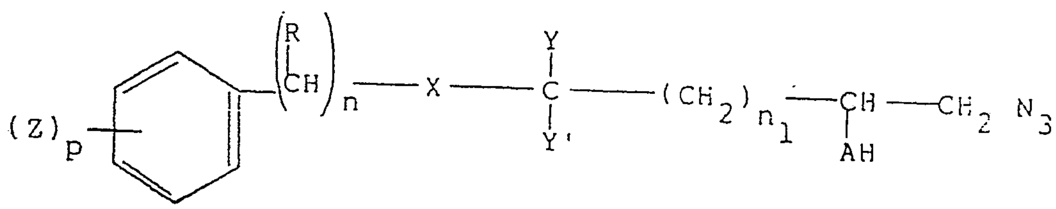

dans laquelle les substituants R, A', X, Y, Y', Z, n, $n_1$ et p sont définis comme précédemment
soumet celui-ci à l'estérification lorsque A' représente de l'oxygène, à l'aide d'un dérivé fonctionnel d'acide sulfonique de formule générale VI

$$R_2\ SO_2\ R_5 \qquad (VI)$$

dans laquelle $R_2$ représente un radical alcoyle ou alcoyle substitué ou un radical aromatique mono- ou bicyclique et $R_5$ est un groupe alcoyloxy inférieur ou un atome d'halogène en présence d'une base tertiaire, pour obtenir un azidoester de formule générale VII

(VII)

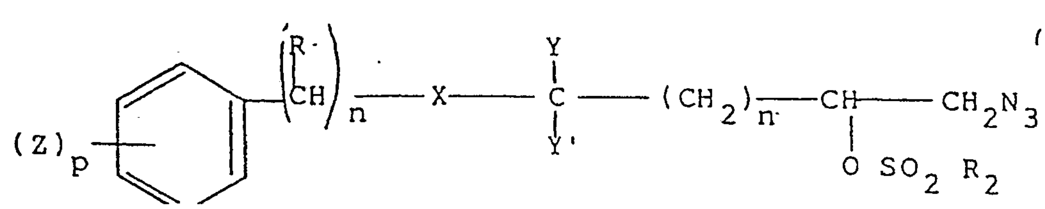

dans laquelle les substituants X, Y, Y', Z, n, p et $R_2$ sont définis comme précédemment
traite celui-ci par un azidure ou un thiocyanate de métal alcalin dans un solvant polaire pour former le dérivé azido de formule générale VIII

(VIII)

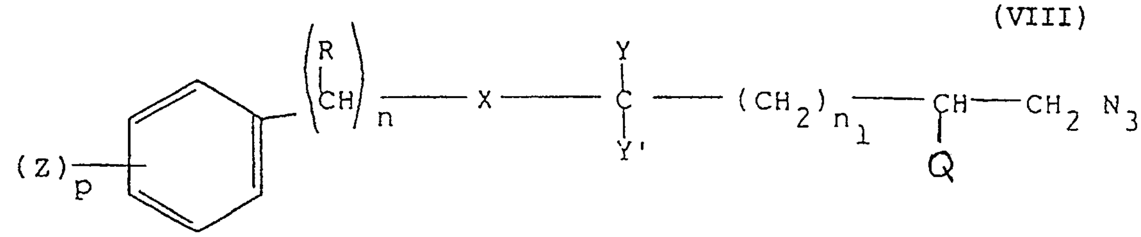

dans laquelle les définitions des substituants R, X, Y, Y', Z, n, $n_1$ et p demeurent inchangées et Q est un radical SH ou azido que l'on réduit par hydrogénation en présence d'un catalyseur en aminoethane de formule générale IX

(IX)

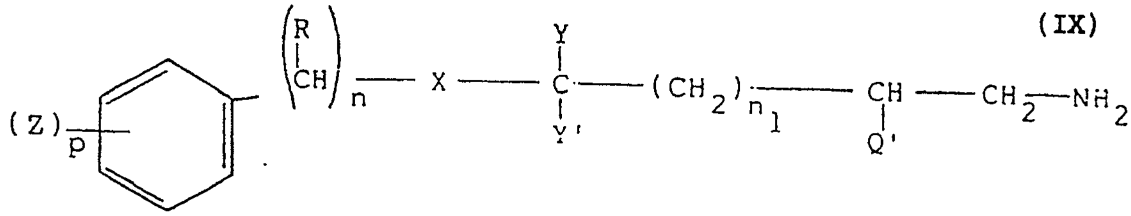

dans laquelle les substituants R, X, Y, Y', Z, n, $n_1$ et p sont définis comme précédemment et Q' est un radical SH ou amino condense celui-ci avec un réactif de carboimination choisi dans le groupe constitué par les halogénures de Cyanogène et les halogénures de S-méthyl isothiouronium de formule générale

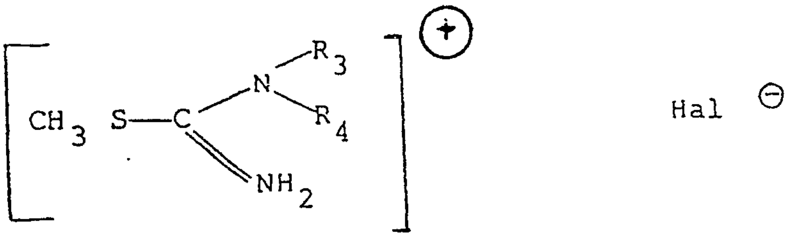

pour obtenir un composé de formule générale I dans laquelle R, X, Y, Y', Z, n et p ont les significations fournies antérieurement A représente du soufre ou un radical imino $R_3$ et $R_4$ représentent tous deux de l'hydrogène lorsque le réactif de carboimination est un halogénure de cyanogène ou $R_3$ et $R_4$ représentent un hydrogène et/ou un radical alcoyle inférieur, alcényle inférieur, aralcoyle inférieur défini comme précédemment, (hétéroaryl) alcoyle inférieur défini comme précédemment, alcoyloxy carbonyle ou acyle dérivé d'un acide organique carboxylique ayant de 1 à 10 atomes de carbone ou bien $R_3$ et $R_4$ forment avec l'atome d'azote une chaine alcoylène lorsque le réactif de carboimination est un halogénure de S-méthyl isothiouronium que l'on peut, si désiré, salifier par addition d'un acide minéral ou organique ou dédoubler en ses isomères optiques par action d'un réactif chiral.

**12.** Un procédé de préparation des composés de formule générale I selon la revendication 1 qui consiste à condenser un composé aromatique de formule générale V avec un halogénure d'allyle pour former un dérivé alcenylé de formule générale X

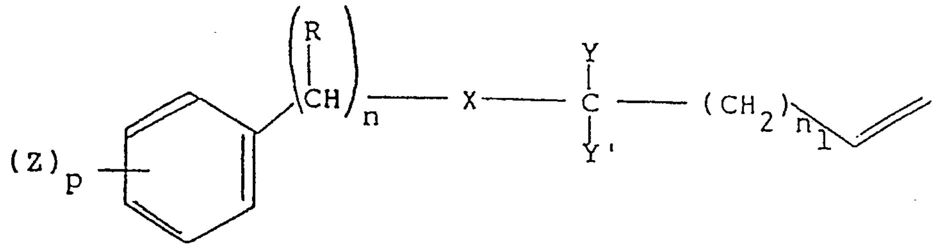

(X)

que l'on soumet à une bromation par action du brome pour former un dérivé dibromé de formule générale XI

(XI)

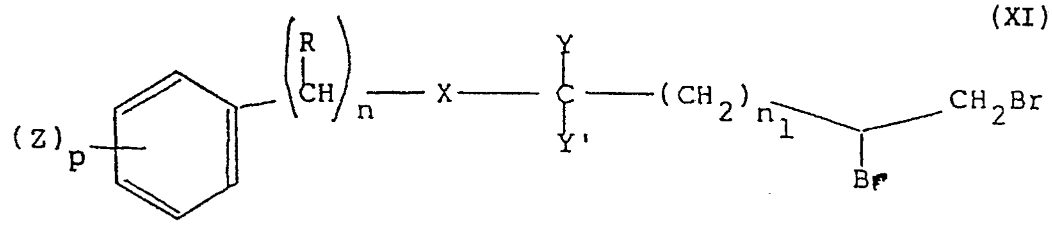

puis fait réagir ce dernier avec un azidure de métal alcalin pour obtenir un diazide de formule générale VIII

(VIII)

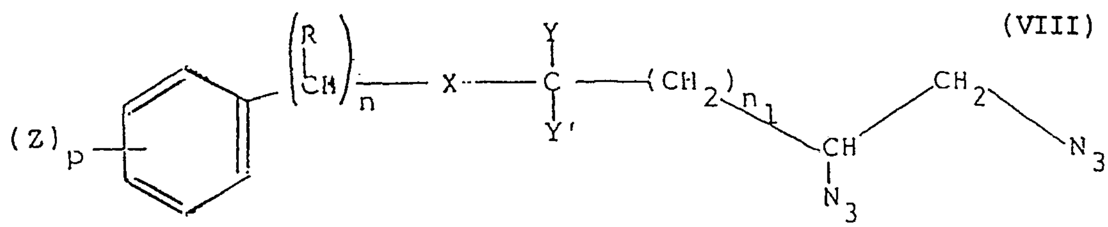

que l'on réduit en dérivé diaminé de formule générale IX par hydrogénation en présence d'un catalyseur
qui fournit par cyclisation sous l'action d'un agent de carboimination, un dérivé imidazolique de formule générale $I_A$

($I_A$)

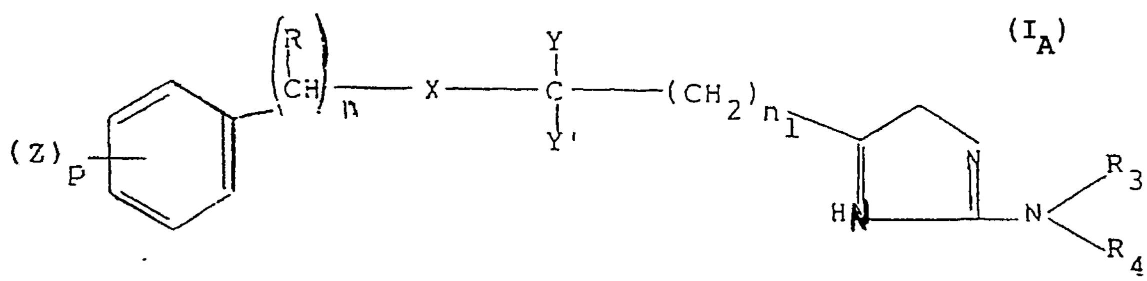

dans laquelle la définition des substances R, Z, X, Y, Y', $R_3$, $R_4$, n, $n_1$ et p demeure inchangée.

**13.** Un procédé de préparation des composés de formule générale I, qui consiste en ce que l'on condense une aryloxyalcoylaldehyde de formule générale XII

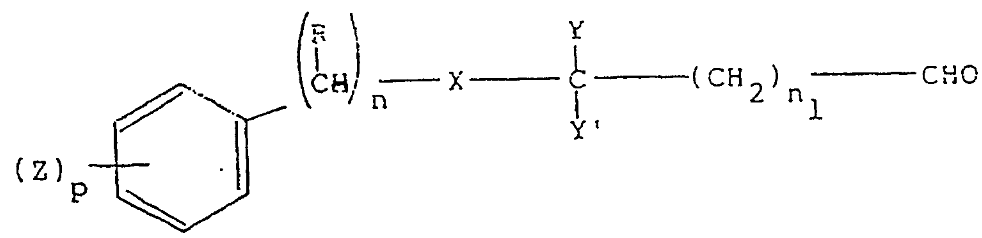

(XII)

dans laquelle la définition des substituants R, X, Y, Y', Z, p, n et $n_1$ demeure inchangée avec un cyanure de métal alcalin dans les conditions de la réaction de Strecker pour former une α-cyano amine de formule générale XIII

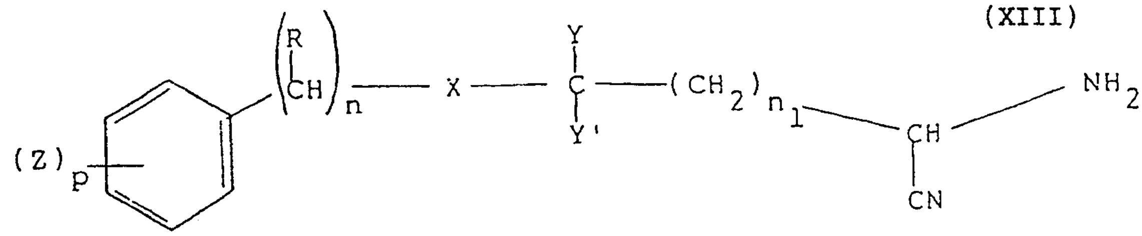

(XIII)

dans laquelle les substituants R, Z, Y, Y', n, $n_1$ et p sont définis comme précédemment qui est réduite par hydrogénation en présence d'un catalyseur en diaminoéthane substitué de formule générale IX

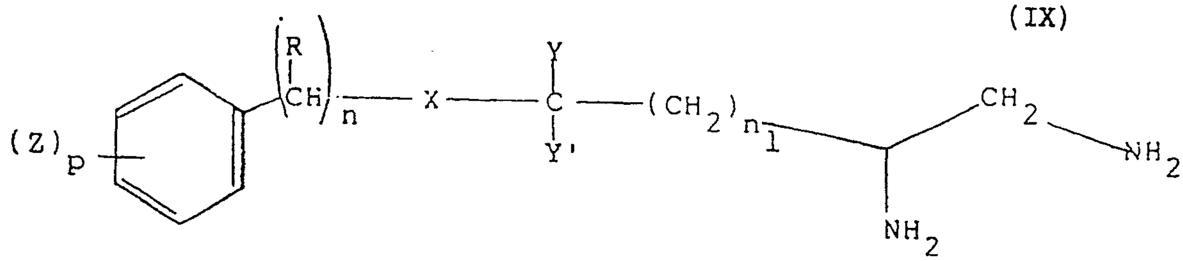

(IX)

dans laquelle la définition des substituants R, X, Y, Y', Z, n, $n_1$ et p demeure inchangée qui par condensation avec un réactif de carbo-imination fournit le dérivé cyclique de formule générale $1_A$

14. Les compositions pharmaceutiques contenant à titre de principe actif au fins un composé de formule générale I, selon la revendication 1 ou un de ses sels d'addition avec un acide minéral ou organique en association ou en mélange avec un excipient ou un véhicule inerte non-toxique pharmaceutiquement acceptable.

15. Les compositions pharmaceutiques selon la revendication 14 qui contiennent en outre un autre principe actif d'action synergique ou complémentaire

16. Une composition pharmaceutique selon l'une des revendications 14 ou 15 dans laquelle la teneur en principe actif varie de 1 à 500 mg par prise unitaire.

## Claims

1. The compounds having the general formula I

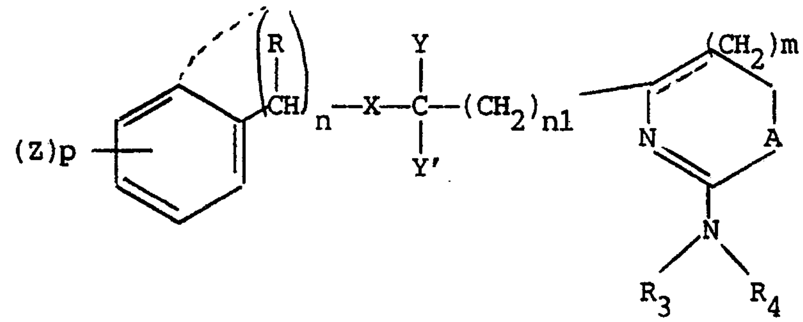

(I)

wherein Z is a hydrogen, a halogen, a lower alkyl radical, a lower alkoxy radical, a trifluoromethyl radical, a trifluoromethoxy radical, a cyano group, a nitro, a carboxamido, a lower alkenyl radical, a (lower alkyl) thio radical, an alkylene dioxy group, a (lower cyclo alkenyl) radical or a (lower cyclo alkyl)-radical

X is a oxygen, a sulphur, an imino group of formula NH>, a direct bond or a methylene radical

Y is a hydrogen, a lower alkyl radical, a phenyl, a hydroxy or a phenoxy

Y' means a hydrogen or

Y and Y' together form the oxygen of a carbonyl group

or Y, together with the adjacent phenyl, form when n is zero, a bicyclic heterocyclic structure selected from the group consisting of tetrahydro indole, dibenzothiophene and thiachroman

A is a NH group or a sulphur atom

$R_3$ and $R_4$, the same or different, represent a hydrogen, a lower alkyl radical, a lower alkenyl radical, an aryl lower alkyl radical the hydrocarbon chain of which has from 1 to 6 carbon atoms, and is straight or branched, selected from the group consisting of a benzyl radical, a phenethyl radical, a $\alpha$-methyl phenyl ethyl, a 2,6-dichorobenzyl, and a 2,3,5-trimethoxybenzyl; a (hetero aryl) lower alkyl radical in which the hydrocarbon chain has from 1 to 6 carbon atoms selected from the group consisting of (pyridyl-2) methyl, furylethyl, pyranylethyl, and (thienyl-2) methyl: an alkoxy carbonyl, an acyl residue from an organic carboxylic acid having from 1 to 10 carbon atoms, an amino group

or $R_3$ and $R_4$ together with the nitrogen atom to which they are bound, form the alkylene chain of a nitrogenous heterocyclic structure which may be optionally interrupted by one or two further hetero atoms

R is a hydrogen, a lower alkyl radical, a phenylene radical linked to the benzene ring by an alkylene chain having 1 or 2 carbon atoms

n is equal to zero or 1

$n_1$ is equal to zero, 1 or 2

m is equal to zero, 1,2 or 3 the dotted line means an optional carbon-carbon double-bond, being understood that the lower alkenyl radical has from 2 to 6 carbon atoms, the lower alkoxy radical has from 1 to 6 carbon atoms and the alkylene dioxy radical has from 1 to 4 carbon atoms.

**2.** The acid addition salts of the compounds according to claim 1, with a mineral or organic acid.

**3.** The tautomeric forms of the compounds according to any of claims 1 and 2.

**4.** The optically-active forms of the compounds according to any of claims 1 to 3.

**5.** The compounds according to claim 1 which have the formula I"

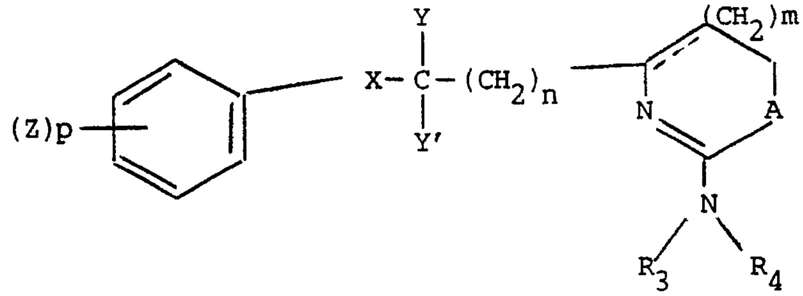

(I")

which correspond to the case wherein n is equal to zero and in which X represent oxygen, sulphur, an imino group of the formula >NH, a methylene group or a direct bond.

**6.** The compounds according to claim 1 having the general formula I'''

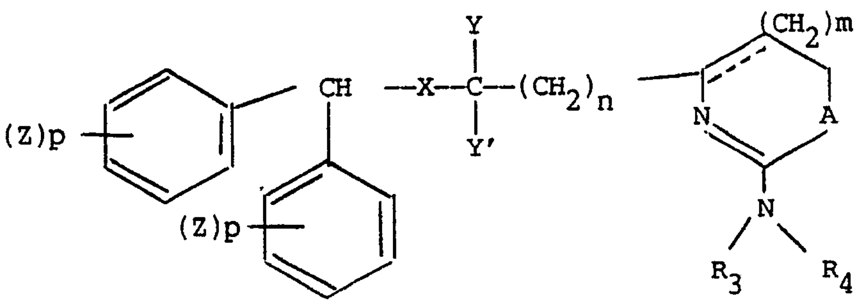

(I''')

wherein the definitions of the substituents X, Y, Y', Z, A, $R_3$, $R_4$, $n_1$ and p are those previously given in claim 1.

**7.** The compounds according to claim 1 having the general formula I""

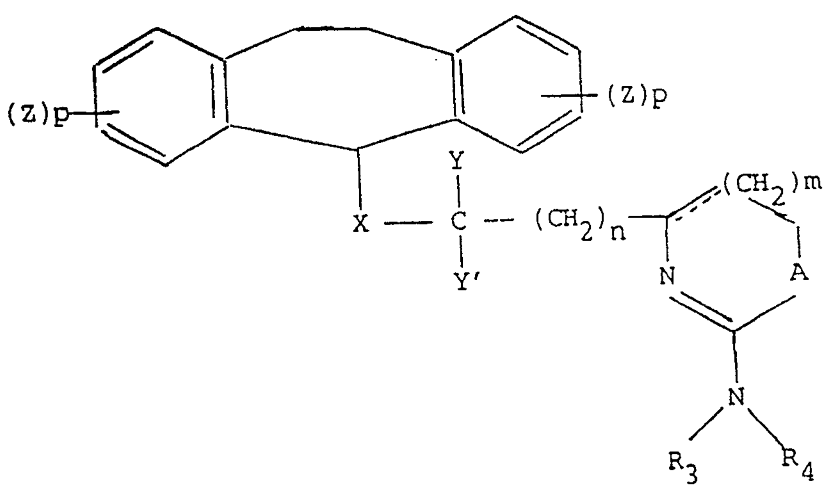

(I"")

wherein the definitions of the substituants X, Y, Y', Z, A, $R_3$, $R_4$, n and p are the same as those provided in claim 1.

**8.** The imidazolines of general formula $I_A$ according to claim 1

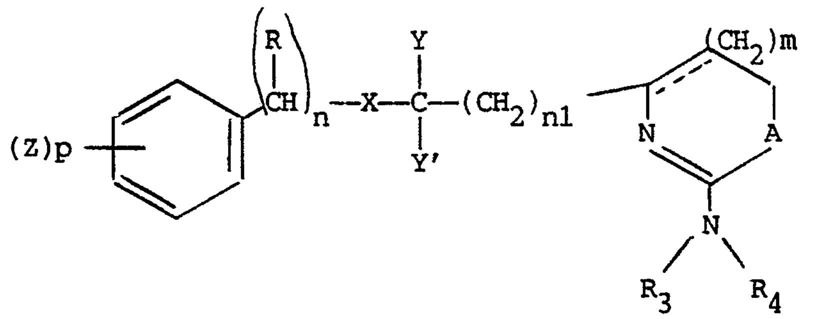

$(I_A)$

in which the definitions of the substituents X, Y, Y', Z, $R_3$, $R_4$, n and p are those previously-given and m is equal to 0.

**9.** The tetrahydropyrimidines of general formula $I_B$ according to claim 1

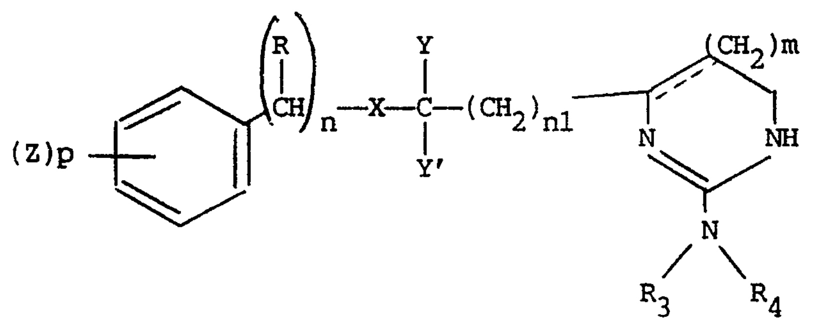

(I)

wherein the substituants X, Y, Y', Z, $R_3$, $R_4$, $n_1$ and p have the above-given definitions and m is equal to 1

**10.** The 2-amino thiazoles of general formula $I_C$ according to claim 1

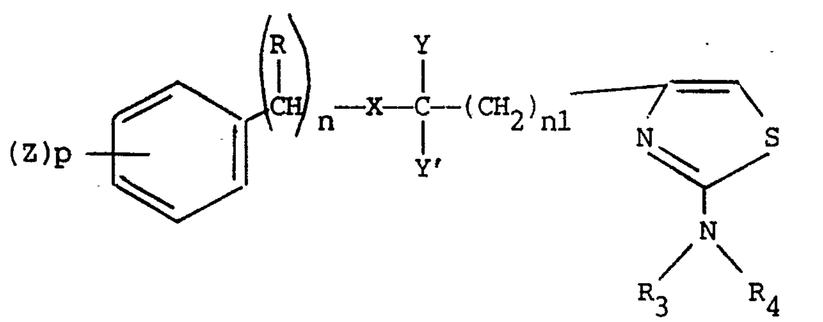

$(I_C)$

wherein the substituents X, Y, Y', Z, R, $R_3$, $R_4$, n, $n_1$ et p have the definitions previously given in claim 1

**11.** A process for producing the compounds of general formula I according to claim 1 wherein m is equal to zero, which consists in that one condenses a epihalohydrin of general formula II

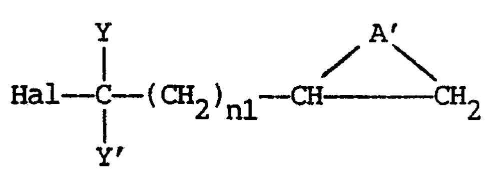

(II)

wherein Hal is a chlorine or bromine

A' is oxygen or a sulphur and the substituents Y, Y' and $n_1$ have the above-given definitions
and m is equal to zero
with an aromatic derivative of general formula III

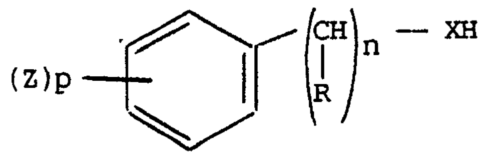

(III)

wherein the substituents R, Z, n, p, and X are defined as above to obtain an arylalkylated derivative of general formula IV

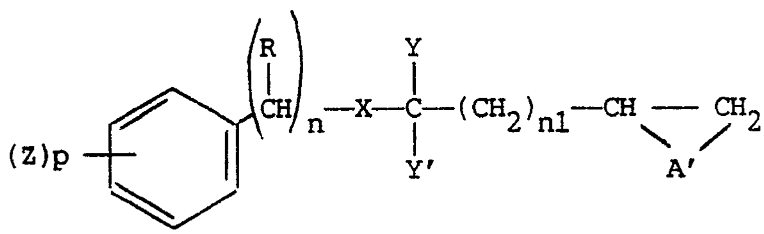

(IV)

wherein the definition of the substituents R, A', X, Y, Y', Z, n, $n_1$ and p remain unaltered
the oxiran ring of which is opened by means of an alkali metal azide to produce a monoazide of general formula V

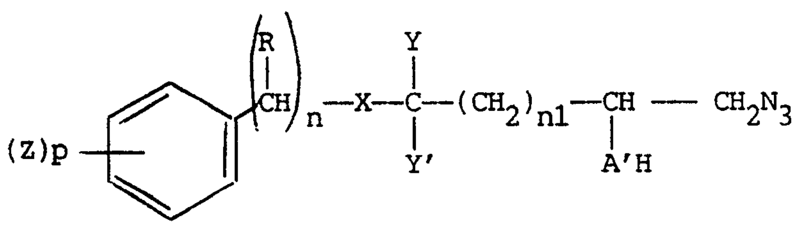

(V)

wherein the substituents R, A', X, Y, Y', Z, n, $n_1$ and p are defined as previously
submits the latter to an esterification when A' is oxygen, by means of a functional derivative of a sulphonic acid having the general formula VI

$$R_2\ SO_2\ R_5 \qquad (VI)$$

in which $R_2$ is an alkyl radical or a substituted alkyl radical or a mono- or bicyclic aromatic radical
and $R_5$ is a lower alkoxy radical or a halogen atom in the presence of a tertiary base to produce an azido ester of general formula VII

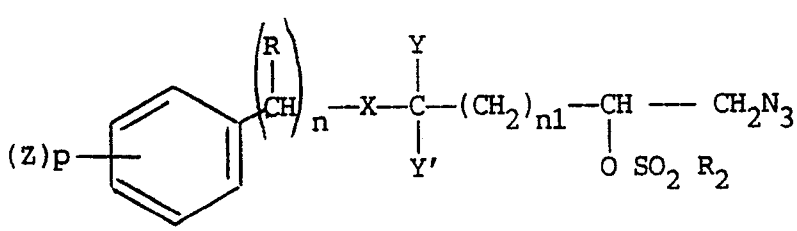

(VII)

in which the substituents X, Y, Y', Z, n, p and $R_2$ are defined as previously
contacts the latter with an alkali metal àzide or thiocyanate in a polar solvent, to produce an azido derivative of general formula VIII

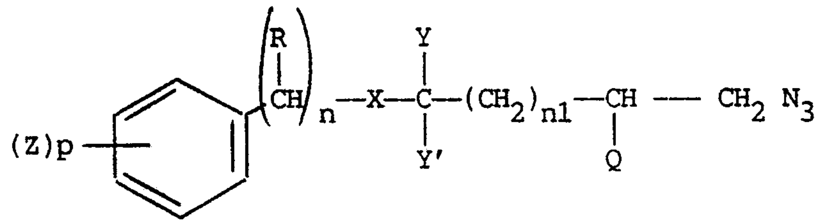

(VIII)

wherein the definitions of the substituent R, X, Y, Y', Z, n, $n_1$ remain unaltered and Q is an azido or thio radical
which is reduced by hydrogenation in the presence of a catalyst, into an amino ethane derivative of general formula IX

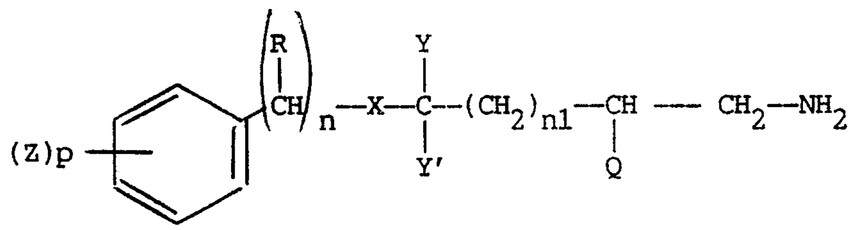

(IX)

in which the substituents R, X, Y, Y', Z, n, $n_1$ and p are defined as previously
and Q is an amino- or SH grouping
condensates the latter with a carboiminating reagent selected from the group consisting of cyanogen halides and S-methyl isothiouronium halides of general formula

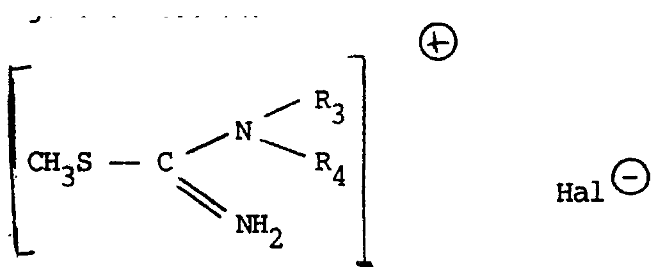

to produce a compound of general formula I

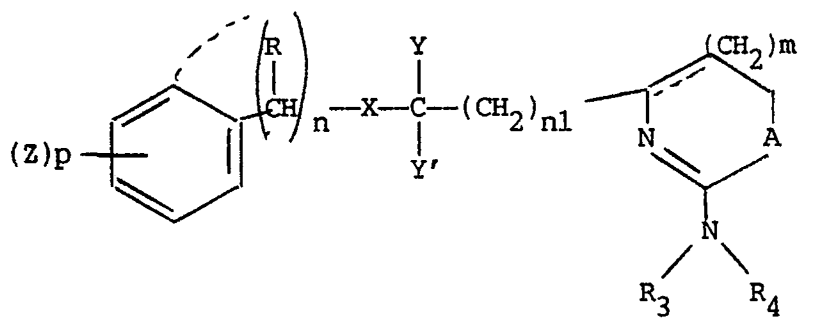

(I)

in which R, X, Y, Y', Z, n and p have the previously-given definitions
A is a sulphur or an imino group
$R_3$ and $R_4$ are both a hydrogen when the carboiminating reagent is a cyanogen halide
or $R_3$ and $R_4$ is a hydrogen and/or a lower alkyl radical, a lower alkenyl radical, an aryl lower alkyl radical defined as previously, a heteroaryl lower alkyl radical defined as previously, an alkyloxy carbonyl or an acyl derivated from an organic carboxylic acid having from 1 to 10 carbon atoms
or $R_3$ and $R_4$ together form with the nitrogen atom, an alkylene chain when the carboiminating reagent is a S-methyl isothiouronium halide which may, if desired, be salified by adding a mineral or organic acid or be resolved into its optical isomers by action of a chiral reagent.

**12.** A process for producing the compounds of general formula I according to claim 1 which consists in condensing an aromatic derivative of general formula V, with an allyl halide to produce an alkenylated derivative of general formula X

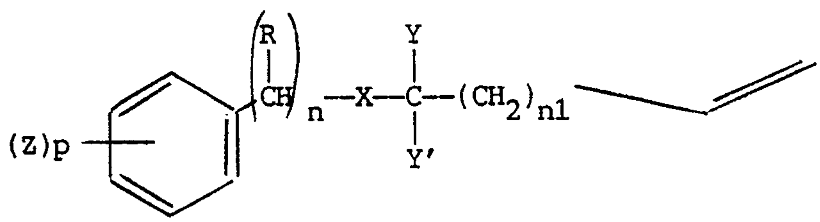

(X)

which is subjected to bromination by means of bromine, to produce a dibrominated derivative of general formula XI

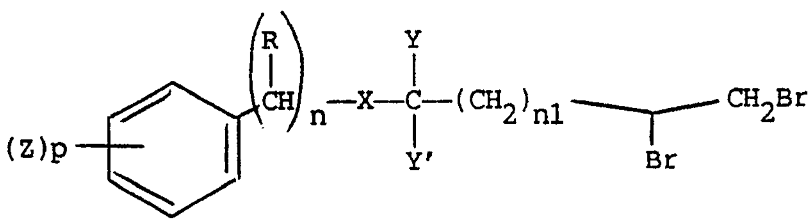

(XI)

then let the latter react with an alkali metal azide to obtain a diazide of general formula VIII

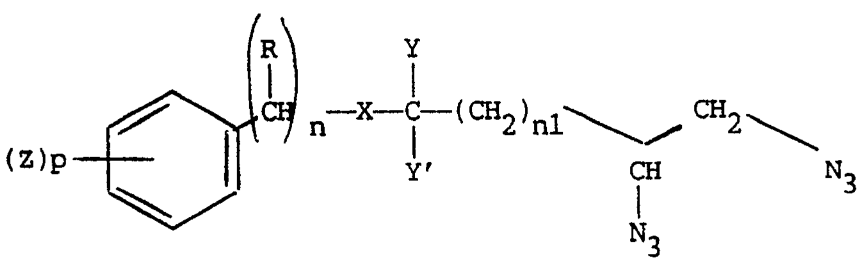

(VIII)

which is reduced into a diamino derivative of general formula IX by hydrogenation in the presence of a catalyst
which provides by cyclization by means of a carboiminating reagent, an imidazolic derivative of general formula $I_A$

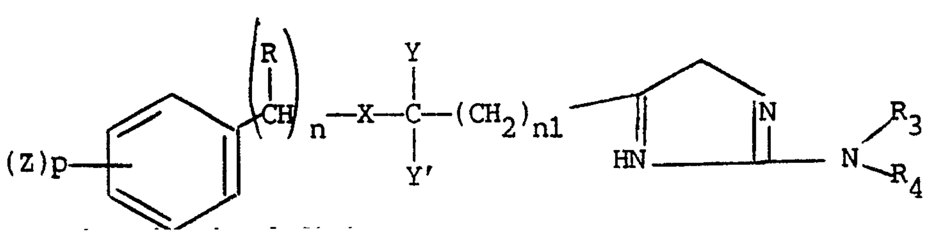

($I_A$)

wherein the definition of the substituents R, Z, X, Y, Y', $R_3$, $R_4$, n, $n_1$ et p remain unaltered.

**13.** A process for producing the compounds of general formula I which consists in condensing an aryloxy alkyl aldehyde of general formula XII

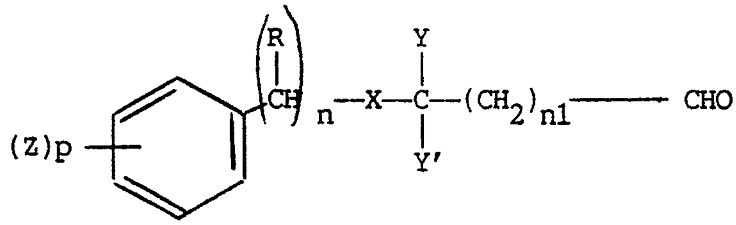

(XII)

wherein the definitions of the substituents R,X, Y, Y', Z, p, n and $n_1$ remain unaltered with an alkali metal cyanide under the conditions of the Strecker's reaction, to produce an $\alpha$-cyano amine of general formula XIII

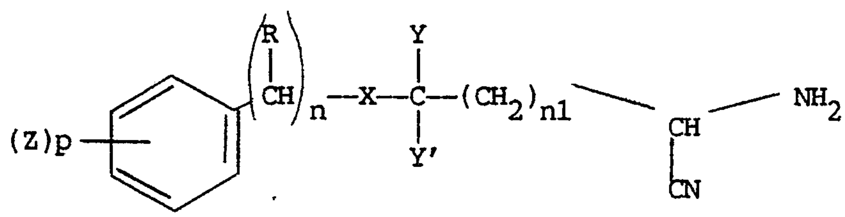

(XIII)

in which the substituents R, Z, Y, Y', n, $n_1$ and p are defined as previously which is reduced by hydrogenation in the presence of a catalyst into a substituted diamino ethane of general formula IX

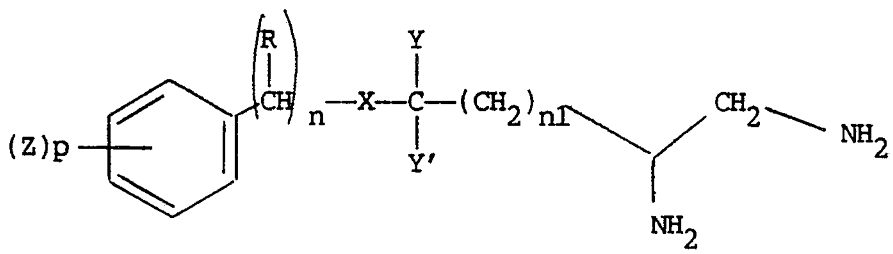

(IX)

wherein the definitions of the substituents R, X, Y, Y', Z, n, $n_1$ and p remain unaltered which after condensation with a carboiminating reagent,provides the cyclic derivative of general formula $I_A$.

14. The pharmaceutical compositions containing as active ingredient at least one compound of general formula I according to claim 1, or an acid addition salt thereof with a mineral or organic acid,in addition or admixture with a carrier or a vehicle which is inert, non-toxic, pharmaceutically-acceptable.

15. The pharmaceutical compositions according to claim 14 which further contain another active ingredient having a synergistic or complementary action.

16. A pharmaceutical composition according to any of the claims 14 or 15 in which the content into the active ingredient ranges from 1 to 500 mg per unit dosage.

## Ansprüche

1. Die Verbindungen die die allgemeinen Formel I besitzen

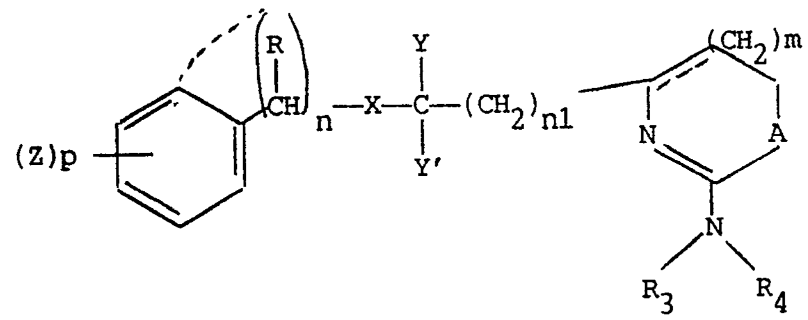

(I)

worin Z ein Wasserstoffatom, ein Halogen, eine niedrig Alkylgruppe, eine niedrig Alkoxy gruppe, eine Trifluoromethyl gruppe, eine Trifluoromethoxy gruppe, eine Cyan gruppe, eine Nitro, ein Carboxamido, eine niedrig Alkenyl gruppe, eine (niedrig Alkyl) thio gruppe, eine Alkylenedioxy gruppe, eine niedrig Cycloalkenyl gruppe, eine niedrig Cycloalkyl gruppe ist

X ein Sauerstoff, ein Schwefel, eine Imino gruppe der Formel >NH, ein gerade Link oder eine Methylen gruppe ist

Y ein Wasserstoff, eine niedrig Alkyl gruppe, ein Phenyl, ein Hydroxy oder ein Phenoxy ist

Y' ein Wasserstoff ist oder zusammen mit Y das Sauerstoff einer Carbonyl gruppe bildet

oder Y zusammen mit dem nachbaren Phenyl, wenn n Null ist, eine zwei-ring heterozyclische Struktur bildet, die aus der Gruppe von Tetrahydroindole, Dibenzothiophene, und Thiachroman gewählt ist

A eine NH gruppe, oder ein Schwefelatom ist

$R_3$ und $R_4$, gleich oder verschiedene, ein Wasserstoff atom, eine niedrig Alkyl gruppe, eine niedrig Alkenyl gruppe, eine Aryl niedrig alkyl gruppe, deren Seitenkette von 1 bis 6 Kohlenstoffatomen besitzt und gerade oder verzweigte ist, unter phenethyl, α-methylphenylethyl, 2,6-dichlorobenzyl und 2,3,5-trimethoxybenzyl gewählt ist, eine (Hetero aryl) niedrig alkyl gruppe in welcher die Kohlenwasserstoff-kette von 1 bis 6 Kohlenstoffatomen besitzt, aus der Gruppe bestehend aus (pyridyl-2) methyl, furylethyl, pyranylethyl, und (thienyl-2)-methyl gewählt ist, ein Alkoxy carbonyl, eine Acyl gruppe der von einer organischen Carbonsaüre mit 1 bis 10 Kohlenstoffatomen abgeleitet wird, oder eine Amino gruppe ist

oder $R_3$ und $R_4$ zusammen mit dem Stickstoff atom womit sie gebunden sind, die Alkylene Kette einer Stickstoff-haltigen (hetero zyklische) Struktur bilden, die gegebenenfalls durch ein oder zwei weitere Hetero atomen unterbrechen werden kann

R ein Wasserstoff, eine niedrig Alkyl Gruppe, eine Phenylengruppe die zù der Phenyl gruppe durch eine Alkylenkette mit 1 oder 2 Kohlenstoff atomen gebunden ist, bedeutet

n gleich null, oder eins ist

$n_1$ gleich null, eins oder zwei ist

m gleich null oder eins ist

und p gleich eins, zwei oder drei ist

und die gestrichelte Linie, ein gegebenfalls vorstehende Kohlenstoff-Kohlenstoff Doppelbindung bedeutet verstanden dass eine niedrig Alkenyl gruppe von 2 bis 6 Kohlenstoff Atomen und die Alkylenedioxy gruppe von 1 bis 4 Kohlenstoff atomen enthalten kann.

**2.** Die Saürezusatz Salze der Verbindungen nach Anspruch 1 mit einer anorganischen oder organischen Saüre

**3.** Die tautomerische Formen der Verbindungen nach einer der Ansprüche 1 oder 2

**4.** Die optische-wirksame Formen der Verbindungen nach einer der Ansprüche 1 bis 3

**5.** Die Verbindungen nach Anspruch 1, mit der Formel I"

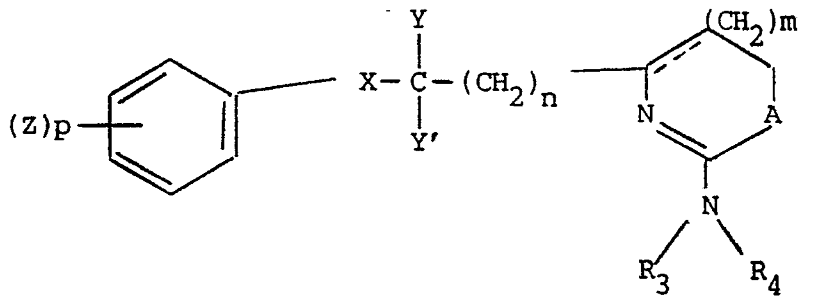

(I")

die den Fall worin n gleich Null ist
und worin Sauerstoff, Schwefel, eine Imino gruppe der Formel >NH, eine Methylen gruppe oder ein gerade Link bedeutet

6. Die Verbindungen nach Anspruch 1, mit der allgemeinen Formel I'''

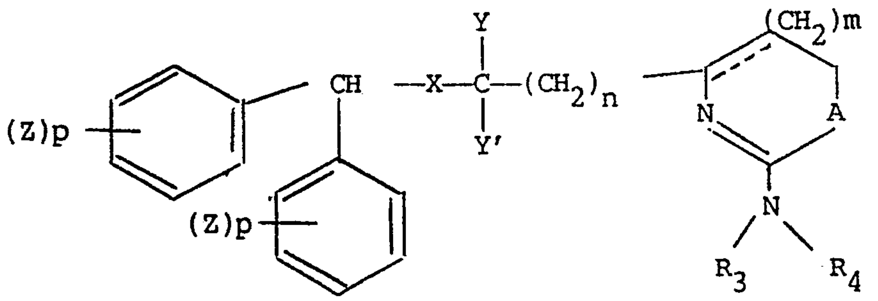

(I''')

worin die Bedeutungen der substituenten X, Y, Y', Z, A, $R_3$, $R_4$, $n_1$ und p dieselbe sind als die vorstehend in Anspruch 1 angegeben sind

7. Die Verbindungen nach Anspruch 1, mit der allgemeinen Formel I''''

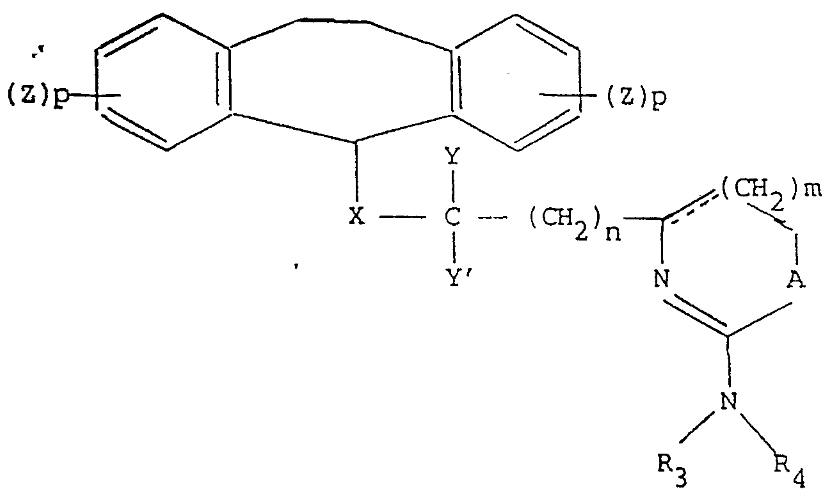

(I'''')

worin die Bedeutungen der Substituenten X, Y, Y', Z, A, $R_3$, $R_4$, n und p dieselbe sind, als die vorstehend in Anspruch 1 angegebene

8. Die Imidazolinen der allgemeinen Formel $I_A$ nach Anspruch 1

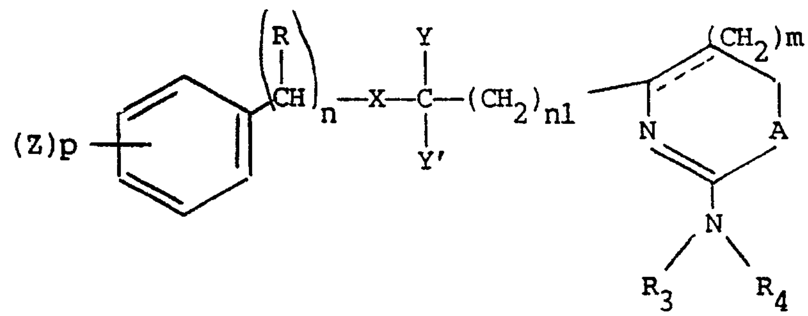

$(I_A)$

worin die Bedeutungen der Substituenten X, Y, Y', Z, $R_3$, $R_4$, n und p dieselbe als vorher, sind und m gleich Null ist

9. Die Tetrahydropyrimidinen der allgemeinen Formel $I_B$ nach Anspruch 1

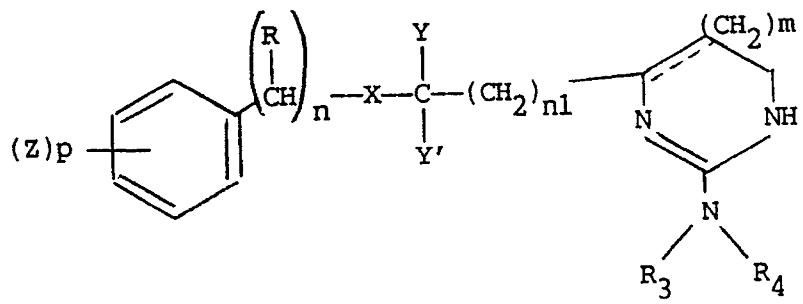

(I)

worin die substituenten X, Y, Y', Z, $R_3$, $R_4$, n und p die vorher angebenene Bedeutungen haben und m gleich 1 ist

10. Die 2-Aminothiazolen mit der allgemeinen Formel $I_C$ nach Anspruch 1

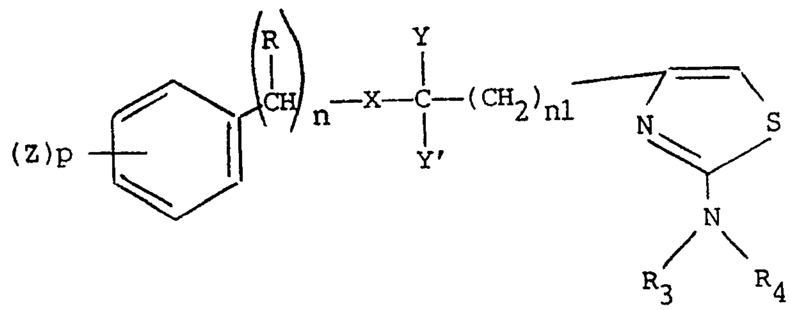

$(I_C)$

worin die substituenten X, Y, Y', Z, R, $R_3$, $R_4$, n, $n_1$ und p die vorher in Anspruch 1 angebenene Bedeutungen haben

11. Ein Verfahren zur Herstellung der Verbindungen der allgemeinen Formel I nach Anspruch 1, worin n gleich Null ist, darin besteht dass man ein Epihalohydrin der allgemeinen Formel II

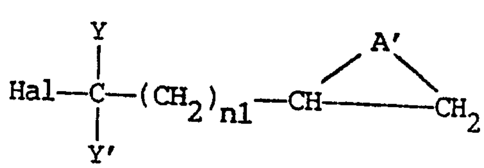

(II)

worin Hal ein Chlor- oder Brom atom ist
A' ein Sauerstoff oder Schwefel bedeutet
und m gleich Null ist

mit einem aromatischen Derivate der allgemeinen Formel III

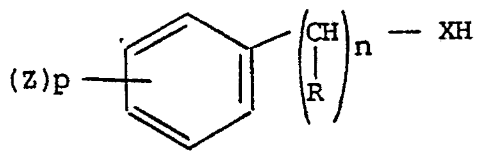

(III)

worin die substituenten R, Z, n, p und X wie vorher gekennzeichnet sind umsetzt, um ein Arylalkyl derivat der allgemeinen Formel IV

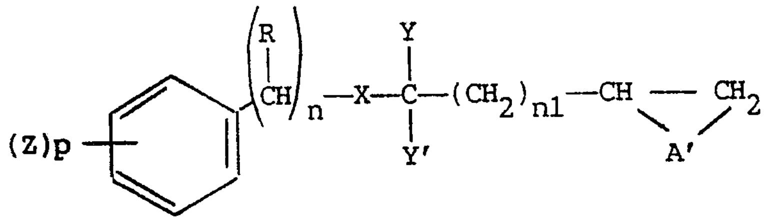

(IV)

worin die Bedeutungen der substituenten R, A', X, Y, Y', Z, n, $n_1$ und p unveränderte bleiben
zu gewinnen, dessen Oxiranring mittels einen Alkalimetal Azid zu einem Monoazid der algemeinen Formel V

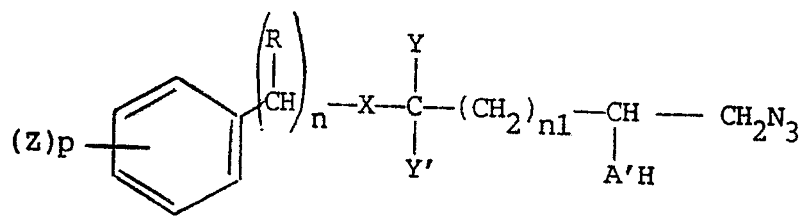

(V)

worin die substituenten R, A', X, Y, Y', Z, n, $n_1$ und p als vorher gekenn-zeichnet sind
geöffnet wird, dieses zu einer Esterifierung, wenn A' ein Sauerstoff ist, mittels eines funktionalen Derivates einer Sulfonsaüre mit der allgemeinen Formel VI

$$R_2\ SO_2\ R_5 \qquad (VI)$$

in dem $R_2$ eine Alkylgruppe, eine substituierte Alkylgruppe oder ein mono- oder bizyclische aromatische Ring ist
und $R_5$ ein niedrig Alkoxy gruppe oder ein Halogen atom ist in Anwesenheit von einem tertiären Base unterwirft, um einen Azidoester der allgemeinen Formel VII

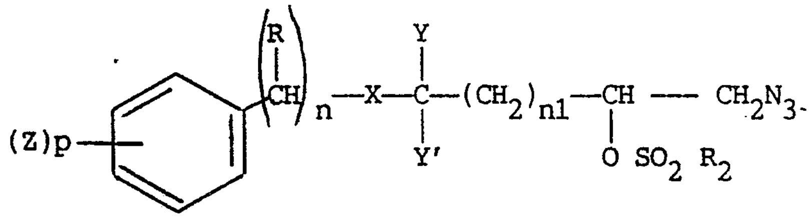

(VII)

zu herstellen
worin die substituenten X, Y, Y', Z, n, p und $R_2$ wie vorher gekennzeichnen sind, dass dieses mit einem Alkalimetallazid oder -Thyocyanat in einem polaren Lösungsmittel zu einem Azido derivate der allgemeinen Formel VIII

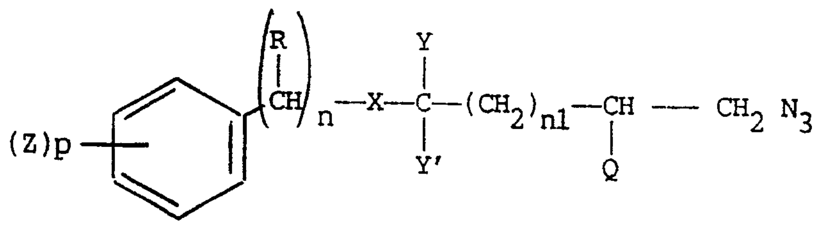

(VIII)

worin die Bedeutungen der substituenten R, X, Y, Y', Z, n, $n_1$ un veränderte bleiben, und A ein Azid- oder Thio gruppe ist überführt, dass dieses durch Hydrierung in Anwesenheit eines Katalysators, zu einem Amino-ethan Derivate der allgemeinen Formel IX

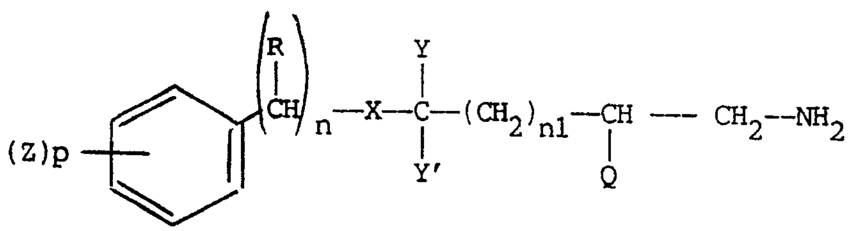

(IX)

worin die substituenten R, X, Y, Y', Z, n, $n_1$ und p als vorher gekenn zeichnet sind und Q eine Amino- oder SH-gruppe ist überführt, dass man dieses mit einem Carboiminierung Reagenz aus der Gruppe aus Cyanogen Halide und S-methyl ThioUronium Halide der allgemeinen Formel

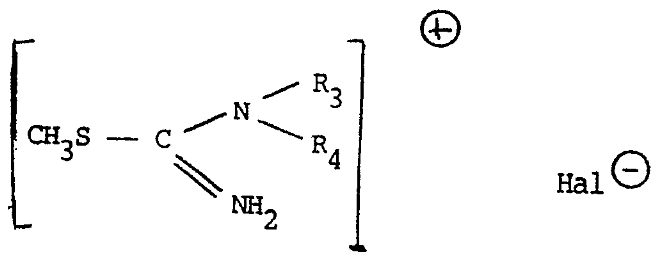

besteht, zyklisiert um eine Verbindung der allgemeinen Formel I

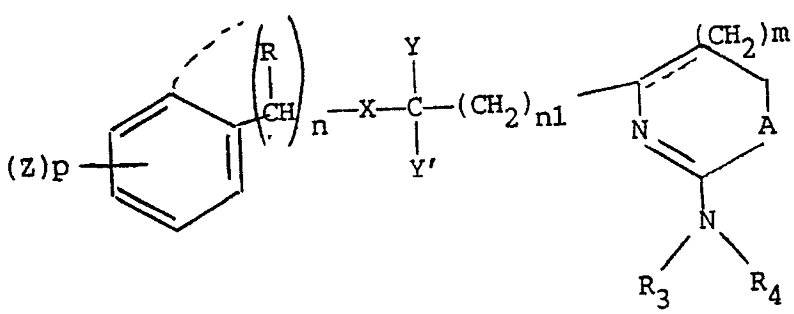

(I)

worin R, X, Y, Y', Z, n und p die vorherige Bedeutungen haben

A ein Schwefel-atom oder eine Imino gruppe ist

$R_3$ und $R_4$, beide, ein Wasserstoff-atom sind, wenn der Carboiminierung Reagenz Cyanogen Halid ist

oder $R_3$ und $R_4$ Wasserstoff und/oder eine niedrig Alkyl gruppe, eine niedrig Alkenyl gruppe, eine Aryl (niedrig alkyl) gruppe als vorher gekennzeichnet, eine Heteroaryl (niedrig alkyl) gruppe als vorher gekennzeichnet, ein Alkyloxycarbonyl, oder die Acyl gruppe einer organischen Carbonsaüre mit von 1 bis 10 Kohlenstoffatomen

oder $R_3$ und $R_4$ zusammen eine Alkylenkette mit den Stickstoff-atom bilden wenn der Carboiminierung Reagenz, ein S-methyl isothioUronium halid ist der gegebenenfalls, wenn gewünscht, in einem Salz mit einer anorganischen oder organischen Saüre überführt kann, oder in seinen optischen Isomeren mittels eines chirales Reagenz spalten kann.

**12.** Ein Verfahren zur Herstellung der Verbindungen der allgemeinen Formel I nach Anspruch 1, darin bestehet dass man ein aromatische Derivate der allgemeinen Formel V mit einem Allyl Halogenid umsetzt um eine Alkenyl Verbindung der allgemeinen Formel X

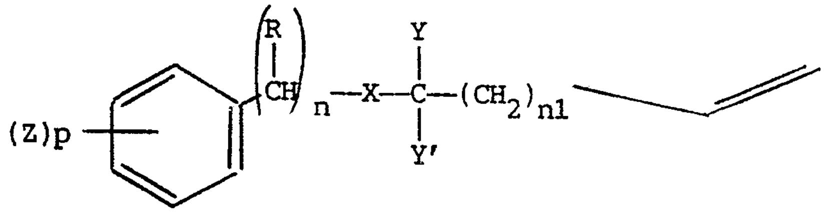

(X)

zu gewinnen
die eine Bromierung, mittels Brom unterwirft um eine Dibromderivate der allgemeinen Formel XI

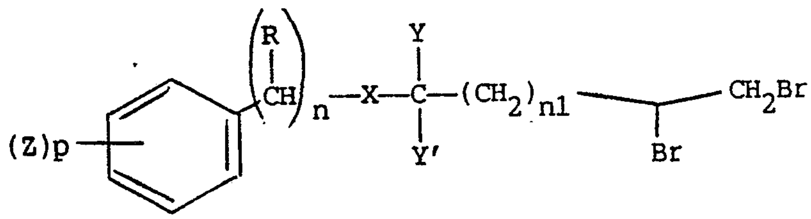

(XI)

zu bilden
dann diese mit einem Alkalimetall Azid umsetzt um ein Diazid der allgemeinen Formel VIII

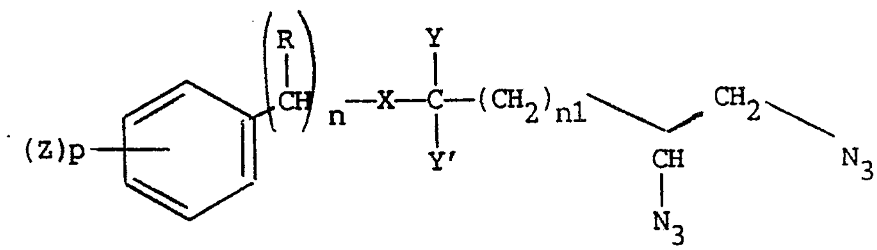

(VIII)

zu erhalten
dass man zu eine Diaminoverbindung der allgemeinen Formel IX durch Hydrierung in Anwesenheit eines Katalysators reduziert, die durch Cyclizierung mittels eines Carboiminierung Reagenz eine imidazolische Derivate der allgemeinen Formel $I_A$

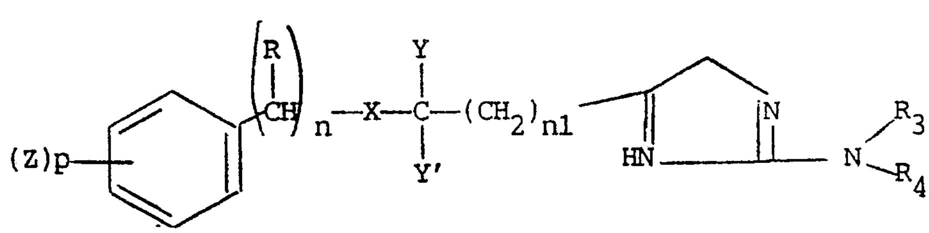

($I_A$)

worin die Bedeutung der substituenten R, Z, X, Y, Y', $R_3$, $R_4$, n, $n_1$ und p unverändert bleibt gibt

**13.** Verfahren zur Herstellung der Verbindungen der allgemeinen Formel I, das darin besteht dass man ein Aryloxyalkylaldehyd der allgemeinen Formel XII

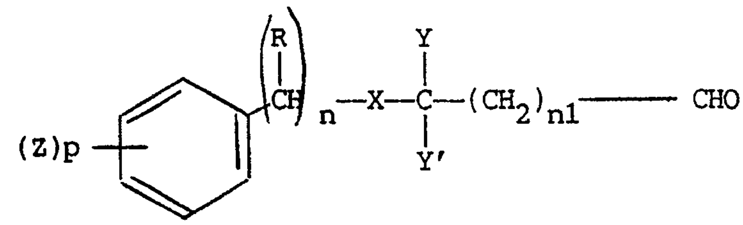

(XII)

worin die Bedeutung der Substituenten R, X, Y, Y', Z, p, n und $n_1$ unverändert bleibt mit einem Alkalimetal Cyanid under den Bedingungen der Streckerschen Reaktion umsetzt um eine α-Cyanoamin der allgemeinen Formel XIII

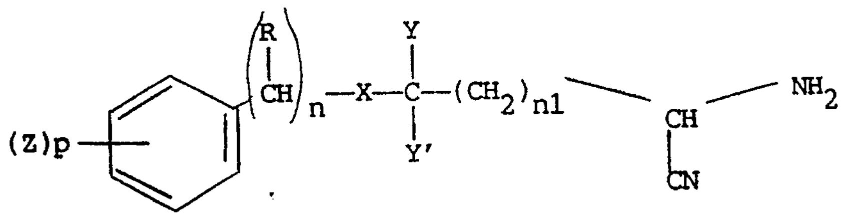

(XIII)

worin die substituenten R, Z, Y, Y', n, $n_1$ und p wie vorher bestimmt sind zu gewinnen, dieses durch Hydrierung in Anwesenheit eines Katalysators zu eine substituierte Diaminoethan der allgemeinen Formel IX

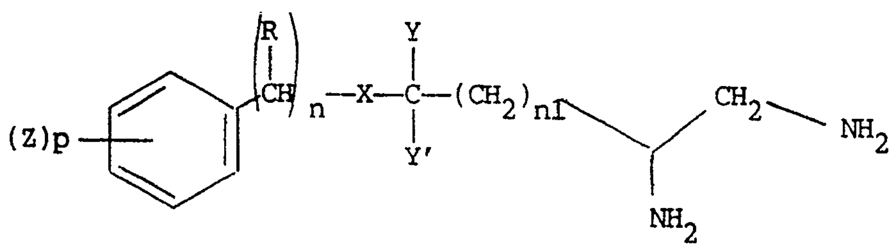

(IX)

worin die Bedeutung der Substituenten R, X, Y, Y', Z, n, $n_1$ und p unverändert bleibt reduziert, das durch Umsetzung mit einem Carboiminierung Reagenz den cyclischen Derivate der allgemeinen Formel $I_A$ ergibt.

**14.** Die pharmazeutische Zubereitungen die als Wirkstoff mindenstens eine Verbindung der allgemeinen Formel I nach Anspruch 1 oder ein der Zusatzsalze mit einer anorganischen oder organischen Saüre, enthält in Verbindung oder Mischung mit einem inerten unbedenklichen, pharmazeutisch verträglichen Träger oder Vehikel

**15.** Die pharmazeutische Zubereitungen nach Anspruch 14 die weiterhin ein andere Wirkstoff mit einer synergistischen oder zusätzlichen Wirkung enthalten.

**16.** Eine pharmazeutische Zubereitung nach Anspruch 14 oder 15 worin die Gehalt an Wirkstoff in dem Bereich von 1 bis 500 mg per Einzelndosis liegt.